(19) Europäisches Patentamt
European Patent Office
Office européen des brevets

(11) **EP 3 174 531 B1**

(12) **EUROPÄISCHE PATENTSCHRIFT**

(45) Veröffentlichungstag und Bekanntmachung des Hinweises auf die Patenterteilung:
**27.11.2019  Patentblatt 2019/48**

(21) Anmeldenummer: **15734576.0**

(22) Anmeldetag: **03.07.2015**

(51) Int Cl.:
**A61K 9/20** (2006.01)

(86) Internationale Anmeldenummer:
**PCT/EP2015/001356**

(87) Internationale Veröffentlichungsnummer:
**WO 2016/015813 (04.02.2016 Gazette 2016/05)**

(54) **DIREKT VERPRESSBARE ZUSAMMENSETZUNG ENTHALTEND MIKROKRISTALLINE CELLULOSE**

DIRECTLY COMPRESSIBLE COMPOSITION CONTAINING MICRO-CRYSTALLINE CELLULOSE

COMPOSITION APTE À LA COMPRESSION DIRECTE ET CONTENANT DE LA CELLULOSE MICROCRISTALLINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.07.2014  EP 14002665**

(43) Veröffentlichungstag der Anmeldung:
**07.06.2017  Patentblatt 2017/23**

(73) Patentinhaber: **Merck Patent GmbH**
**64293 Darmstadt (DE)**

(72) Erfinder:
• OGNIBENE, Roberto
  64297 Darmstadt (DE)
• BAUER, Finn
  64625 Bensheim (DE)
• WEDEL, Thorsten
  64589 Stockstadt/Rhein (DE)
• MODDELMOG, Guenter
  64354 Reinheim (DE)

(56) Entgegenhaltungen:
WO-A1-88/07366     US-A1- 2008 152 595

## Beschreibung

Die vorliegende Erfindung betrifft eine direkt verpressbare Zusammensetzung zur Herstellung von Tabletten, die feinkörnige Polyvinylalkohole (PVAs) mit einer mittleren Partikelgröße $D_{v50}$ im Bereich von 50 bis 260 $\mu$m und feinkörnige mikrokristalline Cellulosen (MCCs) mit mittleren Korngrößen von $D_{v50}<70$ $\mu$m in einer Co-Mischung enthalten. Gegenstand der vorliegenden Erfindung sind auch die Verwendung dieser Mischung und wirkstoffhaltige Tabletten mit verlängerter Wirkstofffreisetzung enthaltend diese Mischung.

### Stand der Technik

Polyvinylalkohole (PVAs) sind synthetische Polymere, welche in verschiedenen Qualitäten insbesondere hinsichtlich ihres Polymerisationsgrads und ihrer Viskosität zur Verfügung stehen. Polyvinylalkohole werden durch alkalische Hydrolyse von Polyvinylacetat gewonnen. Das Polyvinylacetat wiederum erhält man durch eine radikalische Polymerisation aus Vinylacetat. Durch unterschiedliche Kettenlängen und unterschiedliche Hydrolysegrade der eingesetzten Polyvinylacetate kann man Polyvinylalkohole (PVAs) verschiedenster physikalischer Eigenschaften erhalten.
Polyvinylalkohole werden insbesondere als Filmbildner, Haftgele und als Viskositätsmodulatoren in einer Vielzahl von Anwendungsgebieten z.B. Lacke, Papiere, Textilien, Kosmetika sowie in Pharmazeutika einschl. Drug Delivery Systemen, etc. eingesetzt.
In der pharmazeutischen Industrie ist die Verwendung von PVAs besonders interessant in pharmazeutischen Zubereitungen wie z.B. in Ophthalmika, als Filmbildner für überzogene Tabletten, als Bindemittel in Granulaten oder als Beschichtungskomponente in Pflastern sowie auch in Drug Delivery Systemen. Ganz besonders interessant ist die Verwendung verschiedener PVA-Typen in der Formulierung von festen oralen pharmazeutischen Darreichungsformen mit einer verlängerten Wirkstofffreisetzung z.B. in sogenannten "Retardtabletten". In diesen Tabletten liegt der Wirkstoff feinverteilt in einer PVA-Matrix vor.
Nach oraler Einnahme wird eine verzögerte Wirkstofffreigabe aus solchen Polymer-haltigen pharmazeutischen Formulierungen dadurch erzielt, dass die Tabletten sich in Gegenwart von Flüssigkeit, wie im Mund oder Magen-Darmtrakt, nicht direkt auflösen sondern quellen und sich ein Gel bildet aus dem der Wirkstoff erst nach und nach durch Diffusion und langsamer Erosion der Gelmatrix in den Gastrointestinaltrakt freigesetzt wird. Diese verzögerte Wirkstoffabgabe aus der Retardtablette führt wiederum zu einem annähernd konstanten Wirkstoffspiegel im Blut und somit einer verbesserten therapeutischen Wirkung.
Dieses bedeutet, dass durch solche galenisch modifizierten Tabletten es möglich ist, den Wirkstoff aus der Darreichungsform in einer kontrollierten Art und Weise über eine längere Zeit im Körper freizusetzen, um dadurch einen therapeutisch wirksamen Blutspiegel des Medikaments über eine längeren Zeitraum (mehrere Stunden) zu halten.
Die beiden wesentlichen Vorteile solcher retardierten Formulierungen sind - im Gegensatz zu Tabletten mit einer sofortigen Wirkstofffreisetzung nach Einnahme - zum einen die Vermeidung von unerwünschten ggf. auch toxischen Blut/Plasmaspiegeln des APIs (API: Active Pharmaceutical Ingredient) als auch eine Reduzierung der Einnahmefrequenz der Tabletten (z.B. statt dreimal täglich nur noch einmal täglich) und somit eine Verbesserung der sog. "Patienten-Compliance", die wiederum mit einem verbesserten therapeutischen Ergebnis der medikamentösen Behandlung verbunden ist.
Bekannte Polyvinylalkohole, die für die Verwendung in pharmazeutischen Formulierungen nach den verschiedenen Pharmakopoen (Pharmacopoea Europaea, Ph. Eur.; United States Pharmakopeia (USP), und der Japanischen Pharmakopoe (JP bzw. JPE) spezifiziert sind, sind jedoch nicht oder nur unter besonderen Bedingungen direkt durch Druckeinwirkung tablettierbar.
Ein besonderes Problem besteht in diesem Zusammenhang also darin, in einfacher Weise Tabletten herzustellen, die vorwiegend aus entsprechenden PVAs als Wirkstoffträger bestehen, worin der Wirkstoff homogen verteilt ist. Eine direkte Tablettierbarkeit von PVA-haltigen Formulierungen ist üblicherweise nur in Gegenwart von höheren Anteilen weiterer Bindemittel, wie Laktose, und von Schmiermitteln und ggfs. weiterer Additiven zu erzielen. Häufig werden Formulierungen, in denen PVAs als Wirkstoffträger eingesetzt werden, in Gegenwart von wässrigen oder alkoholischen Lösungen hergestellt. Beispielsweise ist es bekannt, entsprechende Tabletten mit verlängerter Wirkstofffreisetzung herzustellen, indem der Wirkstoff und PVA in Gegenwart von weiteren Zusätzen nach einer Nassgranulation komprimiert werden. Letzteres ist mit dem Nachteil verbunden, dass die für die Nassgranulierung erforderlichen Lösungsmittel unter hohem Energieeinsatz wieder entfernt werden müssen.
US 2008/152595 A1 offenbart direkt verpressbare Co-Mischungen, die feinkörnige Polyvinylalkohole und feinkörnige mikrokristalline Cellulosen enthalten.

### Aufgabe der vorliegenden Erfindung

Wie aus dem oben Beschriebenen hervorgeht, werden, um die gewünschten Retardierungseffekte zu erzielen, häufig quellende Polymere als Matrix eingesetzt aus denen nach Befeuchtung z.B. im Magen und Darm der Wirkstoff zeitkontrolliert über Diffusions- und Erosionsvorgänge freigesetzt und zur Resorption zur Verfügung gestellt wird. Polyvinylalkohole (PVAs) kommen üblicherweise zum Einsatz, wenn z.B. Unverträglichkeitsreaktionen zwischen Wirkstoff und den häufig als Retardierungspolymer verwendeten Hydroxypropylmethylcellulosen (HPMCs) bestehen oder wenn die eingesetzten HPMC-Typen ein unbefriedigendes Freisetzungsprofil des Wirkstoffs zeigen.

Zur schnellen Tablettenentwicklung mit Retardierungseffekt benötigt der Galeniker ein quellendes Polymer, welches direkt verpressbar ist und dennoch den Wirkstoff zeitkontrolliert freisetzt. Bekannte pulverförmige PVAs sind per se jedoch nicht direkt verpressbar und liefern Tabletten ungenügender Härten, die sich in der pharmazeutischen Praxis nicht handhaben lassen, da sie beispielsweise eine unerwünschte Bruchneigung oder einen zu hohen Abrieb aufweisen.

Daher sind für die schnelle Entwicklung solcher auf Polyvinylalkoholen basierenden Retardtabletten direkt verpressbare Polyvinylalkohol-Trägermaterialien wünschenswert. Durch solche Trägermaterialien wären langwierige und kostenintensive Granulierungsschritte überflüssig, die normalerweise notwendig sind, um die Tablettiermischungen verpressbar zu machen.

Es ist daher Aufgabe der vorliegenden Erfindung, direkt verpressbare Retardierungsmatrices zur Verfügung zu stellen, die zeitaufwendige Granulierungsverfahren überflüssig machen; d. h. solche Schritte, die beispielsweise aus dem Befeuchten mit Granulierflüssigkeiten, mechanischem Vermischungen in Mischsystemen oder in Wirbelschichtanlagen, sowie Nachtrocknungsverfahren zur Entfernung der Granulierflüssigkeiten und Siebungen bestehen, so dass Zeit und Energie eingespart werden können, aber auch teure und zeitaufwändige Investitionen in spezielle Granulieranlagen. Aufgabe der vorliegenden Erfindung ist es auch, solche vorteilhaften direkt verpressbaren Retardierungsmatrices auf Basis von vorwiegend aus PVAs bestehenden Zusammensetzungen zur Verfügung zu stellen. Darüber hinaus ist es Aufgabe der vorliegenden Erfindung, ein Verfahren zur Verfügung zu stellen, durch das PVAs, bzw. handelsübliche PVA-Qualitäten in einen direkt verpressbaren Zustand überführt werden können.

Kurze Beschreibung der Erfindung

Gegenstand der vorliegenden Erfindung sind direkt verpressbare Co-Mischungen, die feinkörnige Polyvinylalkohole (PVAs) mit einer mittleren Partikelgröße $D_{v50}$ im Bereich von 50 bis 260 $\mu$m und feinkörnige mikrokristalline Cellulosen (MCCs) mit mittleren Korngrößen von $D_{v50}<70$ $\mu$m enthalten und durch die dem Galeniker direkt verpressbare Zusammensetzungen mit retardierter Wirkstofffreisetzung zur Verfügung gestellt werden. Vorzugsweise sind solche Mischungen Gegenstand der vorliegenden Erfindung, worin die eingesetzten feinkörnigen Polyvinylalkohole (PVAs) und feinkörnigen mikrokristallinen Cellulosen (MCCs) die Anforderungen der Pharmakopöen (Ph. Eur., USP/NF und JPE erfüllen. Die Lösung der Aufgabe der vorliegenden Erfindung erfolgt insbesondere durch direkt verpressbare Co-Mischungen, enthaltend feinkörnige mikrokristalline Cellulosen mit mittleren Korngrößen von $D_{v50}<65$ $\mu$m, bevorzugt mit mittleren Korngrößen $D_{v50}<20$ $\mu$m, insbesondere im Bereich von $D_{v50}$ 1 $\mu$m - 20 $\mu$m.

In erfindungsgemäßen direkt verpressbaren Co-Mischungen mit verbesserten Eigenschaften liegen die beschriebenen feinkörnigen Polyvinylalkohole und feinkörnigen mikrokristallinen Cellulosen im Verhältnis von 5 : 1 bis 1 : 5 bezogen auf das Gewicht vor, bevorzugt in einem Verhältnis im Bereich von 2 : 1 bis 1 :2, insbesondere bevorzugt in einem Verhältnis im Bereich von 1 : 1 vor.

Erfindungsgemäß können entsprechende direkt verpressbare Zusammensetzungen feinkörnige Polyvinylalkohole (PVAs) der Typen 18-88, 26-88 und 40-88, die den Pharmakopoen Ph. Eur., JPE und der USP entsprechen und alle dazwischen liegenden Qualitäten, sowie Type 28-99, die der JPE und Ph. Eur. entspricht, enthalten.

Die Lösung der Aufgabe der vorliegenden Erfindung erfolgt insbesondere durch direkt verpressbare Co-Mischungen, enthaltend feinkörnige Polyvinylalkohole (PVAs), welche der Ph. Eur. entsprechen und welche durch Polymerisation von Vinylacetat und durch anschließende teilweise oder nahezu vollständige Hydrolyse des Polyvinylacetats erhalten worden sind. Besonders gut geeignet sind solche feinkörnigen PVAs, die eine durchschnittliche relative Molekülmasse im Bereich zwischen 20 000 und 150 000 g/mol haben, und die nach Ph. Eur. eine Viskosität im Bereich von 3 - 70 mPa.s, (gemessen in einer 4%igen Lösung bei 20 °C) besitzen und eine Esterzahl von nicht größer als 280 mg KOH/g (Hydrolysegrad >72.2 mol%) aufweisen.

Insbesondere sind entsprechende direkt verpressbare Co-Mischungen geeignet, welche Polyvinylalkohole (PVAs) enthalten, bei denen es sich um wasserquellbare Harze handelt, die nach USP durch die Formel

$$(C_2H_4O)_n$$

charakterisiert sind, worin
n eine ganze Zahl im Bereich von 500 und bis 5 000 bedeutet, und die durch eine 85 - 89 %ige Hydrolyse des Polyvinylacetats erhalten worden sind.

Darüber hinaus sind auch wirkstoffhaltige Tabletten mit verlängerter Wirkstofffreisetzung von mehreren Stunden Gegenstand der vorliegenden

Erfindung, und zwar Tabletten, enthaltend eine Co-Mischung aus feinkörnigen Polyvinylalkoholen (PVAs) und feinkörnigen mikrokristallinen Cellulosen (MCCs) wie oben charakterisiert.

Darüber hinaus wurde gefunden, dass wirkstoffhaltige Tabletten, die eine entsprechende direkt verpressbare Co-Mischung in einer Menge von 1 - 99 Gew.-%, bevorzugt in einer Menge von 5-95 Gew.-%, ganz besonders bevorzugt in einer Menge von 10-90 Gew.-% bezogen auf das Gesamtgewicht der Tablette enthalten, die gewünschte, verlängerte Wirkstofffreisetzung aufweisen.

Vorteilhafter Weise können mit solchen Zusammensetzungen bereits bei Einsatz von niedrigen Presskräften Tabletten mit besonders hohen Tablettenhärten erhalten werden, die im Herstellungsprozess überraschend geringe Ausstoßkräfte erfordern, und die nur geringe Friabilitäten von ≤0.2 Gew.-% aufweisen.

Bereits bei Verwendung der erfindungsgemäßen Co-Mischungen durch Einwirkung einer Presskraft von 10 kN Tabletten mit einer Tablettenhärte von ≥153 N erhalten werden mit einer Friabilität von ≤0.2 Gew.-%. Durch Kompression mit einer Preßkraft von 20 kN werden bei Verwendung der erfindungsgemäßen Co-Mischungen Tabletten mit Härten von ≥289N erhalten, die Friabilitäten von ≤0.1 Gew.-% aufweisen.

Besonders gut lassen sich unter Verwendung der beschriebenen Co-Mischungen Tabletten mit verzögerter Wirkstofffreisetzung herstellen, welche vorzugsweise Wirkstoffe der BCS Klasse I enthalten, entweder allein oder in Kombination mit anderen Wirkstoffen. Sofern erwünscht und eine klinische Notwendigkeit besteht, können aber auch Wirkstoffe anderer BCS Klassen mit dem erfindungsgemäßen Verfahren zu direkt verpressbaren Darreichungsformen mit einer retardierten Wirkstofffreisetzung verarbeitet werden.

## Detaillierte Beschreibung der Erfindung

Häufig hängt die ausreichende Wirksamkeit von Arzneimitteln von einer gleichmäßigen Dosierung ab, und erfordert eine mehrmalige Verabreichung am Tag, so dass unerwünschte Nebenwirkungen vermieden werden können. Dieses ist jedoch bezüglich der Patientencompliance nicht erstrebenswert. Daher ist es für die Verabreichung bestimmter Wirkstoffe wünschenswert Tablettenformulierungen zur Verfügung stellen zu können, durch die eine Wirkstofffreisetzung langsam über Stunden verläuft, so dass sich bei regelmäßiger Einnahme ein weitgehend konstanter wirksamer Blutspiegel über den Tag einstellt, aber nur eine einmalige Einnahme pro Tag erforderlich ist.

Abhängig von den einzusetzenden Wirkstoffen sind die Anforderungen an die jeweilige Zusammensetzung unterschiedlich. Je nach ihren chemischen und physikalischen Eigenschaften sind andere Wirkstoffträger und Tablettierhilfsmittel zu verwenden, da nicht jeder Wirkstoff mit jedem Tablettierhilfstoff verträglich ist, bzw. aufgrund der chemischen und physikalischen Eigenschaften nicht miteinander verarbeitet werden können.

Die Bioverfügbarkeit von Wirkstoffen kann nach einem biopharmazeutischen Klassifizierungssystem (BCS) unterteilt werden, das Mitte der 1990er Jahre in den USA durch Gordon Amidon entwickelt worden ist und inzwischen Bestandteil sowohl einer US-FDA (Food and Drug Administration) Richtlinie als auch einer Leitlinie der europäischen Arzneimittelagentur zur Beurteilung der Bioäquivalenz von Arzneimitteln ist.

Beispielsweise sind Wirkstoffe der BCS Klasse I gut lösliche Wirkstoffe mit einem hohen Permeationsvermögen. Ihre Resorption wird nur durch die Geschwindigkeit der Magen- und Darmentleerung kontrolliert. Bei Wirkstoffen, die zu dieser Klasse gehören, deren Wirksamkeit aber über den gesamten Tag erwünscht ist, ist man bestrebt, Formulierungen zu entwickeln, durch die eine verzögerte, gleichmäßige Wirkstoffabgabe ermöglicht wird.

Das biopharmazeutische Klassifizierungssystem (kurz BCS, engl.: Biopharmaceutics Classification System) beschreibt Zusammenhänge, die bei der oralen Applikation von Arzneistoffen eine wichtige Rolle spielen. Das System basiert auf der Arbeit von G. Amidon und Kollegen aus dem Jahre 1995. Die Autoren beschreiben in dieser Arbeit, dass die orale Bioverfügbarkeit von Arzneistoffen hauptsächlich von deren Löslichkeit, der Auflösungsgeschwindigkeit sowie der Permeabilität durch biologische Membranen beeinflusst wird. (Amidon GL, Lennernas H, Shah VP; Crison JR. A theoretical basis for a biopharmaceutic drug classification: the correlation of in vitro product dissolution and in vivo bioavailability. Pharm Res. 1995; 12:413.)

Bei Wirkstoffen der BCS Klasse 1 ist sowohl die Löslichkeit als auch die Permeabilität hoch.

Dieses bedeutet, wenn sowohl die Löslichkeit als auch die Permeabilität des Arzneistoffs hoch sind, kann angenommen werden, dass die Absorptionsrate hauptsächlich durch die Geschwindigkeit der Magen- und Darmentleerung bestimmt wird.

Seit August 2000 findet das BCS-System Anwendung beim Zulassungsverfahren für Fertigarzneimittel der amerikanischen Zulassungsbehörde für Arzneimittel FDA (Food and Drug Administration). Unter bestimmten Voraussetzungen kann bei der Beantragung der Zulassung für Fertigarzneimittel auf Bioverfügbarkeits- und Bioäquivalenzstudien verzichtet werden, wenn durch Anwendung des BCS-Systems nachgewiesen wird, dass das neue Fertigarzneimittel (FAM) und ein bereits zugelassenes FAM desselben Arzneistoffes bioäquivalent sein müssen. Dann kann eine Befreiung (engl.: waiver) von der Verpflichtung beantragt werden, diese kostenintensiven und in diesem Fall unnötigen Studien zur Bioverfügbarkeit durchzuführen. Dazu muss der Arzneistoff in der jeweiligen Arzneiform bestimmte Anforderungen bezüglich der Hauptparameter Löslichkeit, Permeabilität und Lösungsgeschwindigkeit erfüllen.

Löslichkeit:

Die höchste Dosis des Arzneistoffs muss sich in maximal 250 mL eines wässrigen Auflösungsmedium in einem pH-Wert Bereich zwischen pH 1 und pH 7,5 vollständig lösen.

Permeabilität:

Ein Arzneistoff besitzt dann eine hohe Permeabilität, wenn mindestens 90% einer verabreichten Dosis vom Körper aufgenommen werden. Dies muss durch geeignete Daten nachgewiesen werden (z.B. Massenbilanz-Studien).

Lösungsgeschwindigkeit:

Die Arzneiform muss eine schnelle Freisetzung des Arzneistoffs gewährleisten. Dies muss durch geeignete in-vitro Freisetzungstests (entweder Drehkörbchen- oder Rührblattmethode) nachgewiesen werden. Mindestens 85% der entsprechenden Dosis müssen innerhalb von 30 Minuten in drei verschiedenen Freisetzungsmedien (0,1 N HCL, Puffer pH 4,5 und Puffer pH 6,8) freigesetzt werden.

Wie oben beschrieben, ist es Ziel der vorliegenden Erfindung einen gut löslichen Wirkstoff gleichmäßig über Stunden zur Verfügung zu stellen. Die Lösung dieses Problems wurde überraschend durch die Verwendung von polymeren Wirkstoffträgern ermöglicht, wobei letztere in Gegenwart von physiologischen Flüssigkeiten, wie Speichel oder Magen- und Darmsaft, langsam ein Gel bilden und den Wirkstoff langsam und kontrolliert durch Diffusion aus der Tablettenmatrix abgeben.

Hier bieten sich Polyvinylalkohole (PVAs) an, die als synthetische Polymere wasserquellbare Harze sind und ausgezeichnete filmbildende und emulgierende Eigenschaften besitzen und in wässrigen Lösungen ein Gel bilden. PVAs sind nach USP durch die Formel

$$(C_2H_4O)_n$$

charakterisiert sind, worin

n eine ganze Zahl im Bereich von 500 und bis 5 000 bedeutet. Je nach der Molekülgröße dieser Polymere und ihrer chemischen Zusammensetzung variieren ihre Eigenschaften sehr, insbesondere hinsichtlich der Wasserlöslichkeit, aber auch in Bezug auf die Tablettierbarkeit.

PVAs werden aus Polyvinylacetat hergestellt, wobei die funktionellen Acetat-Gruppen teilweise oder vollständig hydrolysiert werden, um funktionelle Alkoholgruppen zu erhalten. In dem Maße, in dem der Grad der Hydrolyse zunimmt, nimmt die Löslichkeit des Polymers in wässrigen Medien zu, aber auch die Kristallinität und die Schmelztemperatur des Polymers nehmen zu. Darüber hinaus variiert die Glasübergangstemperatur, abhängig vom Grad der Hydrolyse. Beispielsweise hat ein 38% hydrolysiertes Material keinen Schmelzpunkt, aber eine Glasübergangstemperatur von etwa 48 °C, wohingegen ein zu 75% hydrolysiertes Material eine Schmelztemperatur von etwa 178 °C, ein zu 88% hydrolysiertes Material einen Schmelzpunkt von ca. 196 °C und ein zu 99% hydrolysiertes Material einen Schmelzpunkt von etwa 220 ° C aufweist, wobei aber das Polymer dazu neigt, sich schnell bei einer Temperatur oberhalb von 200 °C zu zersetzen.

Zur Herstellung der erfindungsgemäßen Zusammensetzungen können Polyvinylalkohole (PVAs) der Typen 18-88, 26-88 und 40-88 und alle dazwischen liegenden Qualitäten, einschließlich des Typs 28-99 gemäß JPE und Ph. Eur. verwendet werden

Obwohl Polyvinylalkohole in Wasser löslich sind, sind sie in fast allen organischen Lösungsmitteln nahezu unlöslich, mit Ausnahme von wenigen Lösungsmitteln, wie beispielsweise in Ethanol mit einer geringfügigen Löslichkeit. Diese Eigenschaften der Polymere machen es sehr schwierig, Tablettenformulierungen herzustellen, in denen PVAs zu einem hohen Anteil enthalten sind und die direkt tablettierbar sind.

Für die Verwendung in pharmazeutischen Formulierungen sind nach den verschiedenen Pharmakopoen Polyvinylalkohole unterschiedlicher Hydrolysegrade spezifiziert.

Die Europäische Pharmacopoeia schreibt vor, dass ein zulässiger Polyvinylalkohol zur Verwendung in pharmazeutischen Dosierungen eine Esterzahl von nicht mehr als 280 und eine mittlere relative Molekülmasse zwischen 20.000 und 150.000 haben muss. Der Prozentsatz der Hydrolyse (H) kann aus der folgenden Gleichung berechnet werden:

$$H = ((100-(0.1535)(EV))/(100-(0.0749)(EV)))x100,$$

wobei EV der Ester-Zahl des Polymers entspricht. Unter der Esterzahl ist die Angabe der Menge Kaliumhydroxid in mg gemeint, die zur Verseifung der Ester in 1 g Probe erforderlich ist. Die Esterzahl berechnet sich aus der Differenz von Verseifungs- und Säurezahl.

Somit sind nach der Monographie des Europäischen Arzneibuches nur PVA-Polymere mit einer prozentualen Hydrolyse von mehr als 72,2% einsetzbar.

Nach der United States Pharmacopeia müssen zur Verwendung in pharmazeutischen Darreichungsformen geeignete Polyvinylalkohole einen prozentualen Hydrolysegrad zwischen 85 und 89% aufweisen und einen Polymerisationsgrad von 500 bis 5000 haben. Der Polymerisationsgrad (DM) wird durch die Gleichung berechnet:

$$DM = (Molar\ Mass)/((86)-(0.42(the\ degree\ of\ hydrolysis)))$$

Ein nach der Europäischen Arzneibuch-Monographie in pharmazeutischen Formulierungen einsetzbares PVA ist ein PVA mit einem Hydrolysegrad zwischen 72,2% und 90%, wodurch sowohl PVAs nach der Ph. Eur. (Hydrolyse von mehr als 72,2%, aber weniger als 90% als auch solche gemäß der USP (Hydrolysegrad zwischen 85 - 89 %) umfasst sind. Diese PVA-Qualitäten haben ein Molekulargewicht im Bereich von 14.000 g / mol bis 250.000 g / mol.

Durch Versuche wurde nun gefunden, dass für eine gute Verarbeitbarkeit in Tablettenformulierungen nicht nur der Hydrolysegrad der eingesetzten Polyvinylalkohole, und damit die Kristallinität, eine Rolle spielt, sondern auch die physikalischen Eigenschaften und Erscheinungsformen der eingesetzten handelsüblichen PVA-Qualitäten.

Wie oben schon angedeutet worden ist, sind Polyvinylalkohole mit entsprechend hohem Hydrolysierungsgrad nur unter besonderen Bedingungen direkt tablettierbar, d. h. es müssen vorab Granulierungsschritte durchgeführt werden oder die eingesetzten PVAs müssen mit weiteren Tablettierhilfsmitteln und leicht komprimierbaren Bindemitteln versetzt werden, so dass der Anteil an Polyvinylalkohol in der Gesamtzusammensetzung verringert ist.

Durch Versuche wurde nun überraschend gefunden, dass besonders feinkörnige Polyvinylalkohole der Direkttablettierbarkeit zugänglich gemacht werden können. Entsprechend feinkörnige Polyvinylalkohole können erhalten werden, wenn geeignete Polyvinylalkohole, die für die Verwendung in pharmazeutischen Formulierungen zertifiziert sind, gemahlen und gesiebt werden.

Auf diese Weise können direkt tablettierbare, PVA-Pulver enthaltende Mischungen hergestellt werden, worin der Gehalt an PVAs überraschend hoch eingestellt werden kann.

Die durchgeführten Versuche haben auch gezeigt, dass die Tablettierbarkeit der so vorbehandelten Polyvinylalkohole weiter verbessert werden kann, indem sie in geeigneter Weise mit weiteren polymeren Hilfsstoffen kombiniert werden. Das heißt, die gemahlenen, feinkörnigen Pulver können anschließend mit weiteren, geeigneten polymeren Hilfsstoffen kombiniert werden, wodurch sich die Kompressibilität der erhaltenen Co-Mischung noch weiter verbessert.

Es hat sich hier gezeigt, dass besonders gut tablettierbare Kombinationen erhalten werden, wenn die gemahlenen, feinkörnigen Polyvinylalkohole mit mikrokristallinen Cellulosen vermischt werden. Hierzu können handelsübliche, mikrokristalline Cellulosen verwendet werden, die für die Verwendung in pharmazeutischen Formulierungen zertifiziert sind, wie beispielsweise die Typen Vivapur® 102 und Emcocel® von JRS Pharma sowie der Type Avicel® PH 102 von FMC Biopolymer. Insbesondere wenn die verwendeten mikrokristallinen Cellulosen besonders feinkörnig sind, zeigt sich eine erheblich verbesserte Kompressibilität der Co-Mischungen.

Dieses ist von besonderer Bedeutung für die Entwicklung von direkt verpressbaren Retardtabletten, da für den Galeniker immer Bedarf nach noch "besseren Hilfsstoffen" besteht, d.h. nach Matrices mit weiter verbesserten Kompressibilitäten. Dieses ist darin begründet, dass angestrebt wird, auch ganz extrem schlecht verpressbare APIs in einem Direkttablettierungsprozess verarbeiten zu können, was jedoch mit einem DC-Material geringerer Kompressibilität nicht gelingt.

Außerdem kann man bei Verwendung einer direkt verpressbaren Tablettiermatrix mit einer verbesserten Komprimierbarkeit deren Einsatzmenge reduzieren, so dass Tabletten geringeren Gewichts und verkleinerter Dimensionen herstellbar sind, wobei die erhaltenen Tabletten auch weiterhin sehr gute Tablettenhärten aufweisen (sog. "Dilution-Effekt"). Diese Eigenschaften sind insbesondere für sog. "hochdosierte" Retardtabletten interessant, da hier die reduzierten Tablettendimensionen das Schlucken durch den Patienten verbessern und somit die Compliance und dadurch den therapeutischen Erfolg gewährleisten.

Überraschenderweise wurde durch die Versuche bei der Prüfung zur Tablettierbarkeit gemahlener PVA-Typen mit verschiedenen mikrokristallinen Cellulosen (MCCs) gefunden, dass es je nach verwendeter MCC-Type zu einer Verschlechterung oder aber zur Verbesserung der Kompressibilität kommen kann. Insbesondere die Typen Avicel PH105, Vivapur 101 und Avicel PH101 bewirken im Vergleich mit anderen MCC-Typen - bei gleichen Presskräften - eine deutliche Erhöhung der Tablettenhärten. Nähere Untersuchungen dieser MCC-Typen zeigten, dass sie sich von den anderen Typen durch ihre Partikelgrößen unterscheiden. Die Partikelgröße dieser MCCs liegen bevorzugt im Bereich von $D_{v50}$: 17 - 67 $\mu$m. Es hat sich gezeigt, je feiner die MCC-Körnung ist desto bessere Tablettenhärten werden in der Kombination mit feinkörnigen PVAs erzielt. Daher sind MCC-Typen mit mittleren Korngrößen kleiner 70 $\mu$m für die

Herstellung der erfindungsgemäßen Co-Mischungen zu verwenden, bevorzugt kleiner 20 μm, gemessen als $D_{v50}$ über Laserbeugung. Bei der Verwendung "grobkörnigerer" MCCs (ab 100 μm und insbesondere ab 180 μm) fallen die Tablettenhärten dagegen deutlich ab.

Als besonders überraschend hat sich in diesem Zusammenhang gezeigt, dass für die Erzielung dieser verbesserten Direktverpressungseigenschaften ganz offensichtlich nur die beschriebenen MCCs geeignet sind; andere üblicherweise die Direktverpressung fördernde Träger, wie z.B. direkt verpessbare Calciumhydrogenphosphate, einschließlich des per se sehr gut direkt tablettierbaren Fujicalins® (Fuji Cemical Industry, Japan), direktverpressbare Sorbitole (z.B. Parteck® SI 400, Merck KGaA, Deutschland), direkt verpressbare Mannitole (z.B. Parteck® M200, Merck KGaA, Deutschland) oder direkt verpressbare Stärken (z.B. Starch 1500, Colorcon Limited, UK), zeigen in der Kombination mit PVAs nicht diese Wirkung und führen nicht zu direkt verpressbaren Pulvermischungen mit den PVAs, wie die eigenen Untersuchungen gezeigt haben.

Durch diesen überraschender Weise gefundenen Effekt kann dem Galeniker nun eine vorwiegend aus PVA und feinkörniger mikrokristalliner Cellulose bestehende, direkt verpressbare Vormischung für die Tablettenherstellung zur Verfügung gestellt werden, die zur Beschleunigung eines Entwicklungsprozesses einer neuen Tablettenformulierung führen kann.

Durch die Verbesserung der Tablettenhärten bei gleichbleibendem PVA/MCC-Verhältnis in der Direktverpressungsmatrix hat der Formulierer nun die Möglichkeit auch bisher nicht oder nur schlecht verpressbare Wirkstoffe in eine Retardtablette zu überführen. Weiterhin ist es ihm nun möglich auch hoch dosierte APIs in eine "patientenfreundliche" Retardtablette mit problemlos schluckbaren Dimensionen zu überführen. Außerdem ist es nun möglich, bei Bedarf bei gleicher PVA-Menge die Menge an mikrokristalliner Cellulose zu verringern und damit das Tablettengewicht und die Tablettendimensionen zu reduzieren ohne den Retardierungseffekt des PVAs zu verändern. Diese Materialien führen zu einem besseren sogenannten "Dissolution"-Effekt als Vergleichsmaterialen, die auf gröbkörnigeren MCC-Typen basieren.

Mikrokristalline Cellulose (MCC) ist eines der wichtigsten Tablettierhilfsmittel in der Pharmazeutikaherstellung und wird bevorzugt als Wirkstoffträger eingesetzt und ist eine wesentliche Komponente für fast jede Art von oralen Dosierungsformen, wie Tabletten, Kapseln, Sachets, Granulate und andere. In Reinform ist mikrokristalline Cellulose (MCC) mit der allgemeinen Formel $(C_6H_{10}O_5)_n$ weiße, freifließende Cellulose in Pulverform, die im Handel mit unterschiedlicher Körnung erhältlich ist. In pharmazeutischer Qualität erfüllt sie die USP Standards. Mikrokristalline Cellulose dient unter anderem als unverdaulicher, nicht resorbierbarer Ballaststoff für kalorienreduzierte Lebensmittel, etwa Salatsoßen, Desserts und Eiscremes, als Trennmittel oder als Trägerstoff. Wie aus der vorhergehenden Beschreibung zu entnehmen ist, dient sie in der Pharmazie als Bindemittel oder Trägerstoff für die Tablettenherstellung. In diesem Zusammenhang hat sie sich als besonders geeignet für die Direkttablettierung erwiesen und führt zu harten, Tabletten, die bei geeigneter Formulierung kurze Zerfallszeiten aufweisen.

MCC wird aus verholzten Pflanzenteilen gewonnen (nicht aus Altpapier). Hierbei wird die Pflanzen-Zellulose mit verdünnter Salzsäure bei Temperaturen über 100° C von nichtkristallinen Zellulose-Anteilen befreit. Das heißt, MCC in Pharmaqualität lässt sich durch partielle Hydrolyse von hoch reiner Cellulose und anschließender Aufreinigung und Trocknung gewinnen. Im Anschluss an die Hydrolyse kann wahlweise eine Carboxylierung erfolgen, um die hydrophilen Eigenschaften zu verbessern.

MCC ist in Wasser, Alkoholen und organischen Lösungsmitteln unlöslich. In Wasser bildet MCC eine dreidimensionale Matrix, bestehend aus unzähligen, unlöslichen Mikrokristallen, die ein stabiles thixotropes Gel ausbilden. Die vorteilhaften Eigenschaften von MCC bleiben auch erhalten bei Temperatur bedingten Änderungen des Phasenzustands, beispielsweise beim Übergang in den Gefrierzustand oder beim Erhitzen auf höhere Temperatur, so dass MCC besonders gut geeignet ist für Fertigmischungen zur Weiterverarbeitung.

Als geeignete MCCs zur Erzielung ausreichenden Tablettenhärten haben sich die handelsübliche Typen erwiesen, die mittlere Partikelgrößen $D_{v50}$ kleiner 70 μm, bevorzugt im Bereich von $D_{v50}$: 17 - 67 μm, insbesondere bevorzugt kleiner 20 μm, gemessen als $D_{v50}$ über Laserbeugung, besitzen. Dabei weisen solche feinkörnigen MCC-Typen vorzugsweise Schüttdichten im Bereich von 0,20 bis 0,35 g/cm³, bevorzugt im Bereich von 0,20 bis 0,31 g/cm³ auf. Geeignete handelsübliche MCC-Qualitäten, die diese Kriterien erfüllen und für die Verwendung in pharmazeutischen Formulierungen qualifiziert sind, sind beispielsweise Vivapur 101 (im Luftstrom getrocknet, mittlere Partikelgröße $D_{v50}$ 65 μm, ermittelt durch Laserdifraktion, Schüttdichte 0,26 - 0,31 g/cm³), Avicel PH 101 (mittlere Partikelgröße 50 μm, Schüttdichte 0,26 - 0,31 g/cm³) sowie Avicel PH 105 (Sprühgetrocknet, mittlere Partikelgröße $D_{v50}$ 20 μm, ermittelt durch Laserdiffraktion, Schüttdichte 0,20 - 0,30 g/cm³). Aber auch andere nicht hier erwähnte Handelsprodukte, die die beschriebenen Erfordernisse erfüllen, können gemäß der hier beschriebenen Erfindung verwendet werden.

Besonders überraschend ist, dass durch Kombination geeigneter mikrokristalliner Cellulosen mit verschiedenen PVA-Qualitäten, insbesondere mit PVAs mit unterschiedlichsten Viskositäten, direkt verpressbare Mischungen erhalten werden, die je nach Bedarf vorwiegend aus PVAs, gegebenenfalls aber auch zu gleichen Anteilen aus PVAs und mikrokristallinen Cellulosen bestehen. Wenn gewünscht, können aber auch Mischungen eingesetzt werden, in denen der Anteil der feinkörnigen mikrokristallinen Cellulosen höher ist als derjenige der feinkörnigen Polyvinylalkohole.

Es hat sich als besonders vorteilhaft erwiesen, wenn in den erfindungsgemäßen Zusammensetzungen das Verhältnis der beschriebenen feinkörnigen Polyvinylalkohole und feinkörnigen mikrokristallinen Cellulosen in einem Bereich 5 : 1 bis 1 : 5 bezogen auf das Gewicht, bevorzugt in einem Verhältnis in einem Bereich von 2 : 1 bis 1:2, insbesondere bevorzugt in einem Verhältnis im Bereich von 1 : 1 liegt. Solche Co-Mischungen haben sich als besonders geeignet erwiesen zur Herstellung von Tabletten mit verzögerter Wirkstofffreisetzung. Nach intensiver Vermischung weisen die hier gefundenen Co-Mischungen aus PVA mit MCCs Schüttdichten im Bereich von 0,38 - 0,48 g/ml bei Stampfdichten im Bereich von 0,53-0,65 g/ml auf.

Durch die beschriebenen vorteilhaften Eigenschaften der Kombinationen aus feinkörnigen Polyvinylalkoholen und feinkörnigen mikrokristallinen Cellulosen erhält der Formulierer in der pharmazeutischen Industrie, aber auch in der Lebensmittelindustrie oder in anderen technischen Bereichen ein Material an die Hand, welches den Entwicklungsaufwand für feste komprimierte Darreichungsformen mit verlängerter Wirkstofffreisetzung deutlich vereinfacht. Er braucht nur noch seinen zu retardierenden Wirkstoff mit der erfindungsgemäßen PVA-MCC-Kombination zu mischen, ggf. einige wenige Hilfsstoffe insbes. Gleit-und Schmiermittel zugeben und diese Mischung dann auf einer Tablettiermaschine verpressen. Durch die besonders guten Tablettiereigenschaften dieser Matrix ist die Entwicklung von Retardtabletten auch mit Wirkstoffen möglich geworden, die per se eigentlich als nicht direkt verpressbar gelten und die in üblicher Weise durchgeführten Prozessen vorher granuliert werden müssten. Die Verwendung dieser PVA-MCC-Matrix spart Entwicklungszeit, Geräteinvestitionen und führt zu einer verbesserten Prozesssicherheit in Entwicklung und Produktion.

Ein vorteilhafter Nebeneffekt stellt sich bei Verwendung der erfindungsgemäßen Co-Mischungen im Tablettierungsprozess ein, der darin besteht dass die erfindungsgemäßen Mischungen zu vergleichsweise niedrigen Ausstoßkräften führen, wodurch es möglich ist, mit deutlich geringeren Mengen an Schmiermitteln zu arbeiten als sonst in der Tablettierung üblich. So werden statt eines üblicherweise 1%igen Magnesiumstearatzusatzes nur noch etwa ein Viertel dieser Menge benötigt, gegebenenfalls auch noch weniger. Unter besonderen Bedingungen kann auch auf den Zusatz von solchen Schmiermitteln gänzlich verzichtet werden. Dies bedingt eine zusätzliche Verbesserung der interpartikulären Bindungskräfte, d.h. es werden härtere Tabletten bei gleicher Presskraft erhalten, wobei eine reproduzierbarere Wirkstofffreisetzung erzielt werden kann. Letzteres ist dadurch begründet, dass die Freisetzung im Wesentlichen über den PVA-Gehalt gesteuert wird und der geringe Zusatz von hydrophobem Magnesiumstearat nur noch einen geringfügigen Einfluss auf das Freisetzungsverhalten ausübt.

Des Weiteren betrifft die vorliegende Erfindung ein Verfahren zur Beeinflussung der Tablettiereigenschaften von feinkörnigen PVA-Qualitäten, insbesondere von gemahlenen PVAs, die an sich nur eine geringe Kompressibiliäten aufweisen. Durch Versuche wurde gefunden, dass diese feinkörnigen PVAs durch Kombination mit feinkörnigen MCCs in eine direkt verpessbare Form überführt werden können.

Feinkörnige PVAs sind zur Verwendung als Retardierungsmatrices besonders geeignet, da sie in der Regel sehr gut eingesetzt werden können, um homogenere Mischungen mit dem zu retardierenden Wirkstoff herzustellen. Letzteres ist für die Einzeldosierungsgenauigkeit "Content Uniformity" von besonderer Bedeutung, um in jeder Einzeltablette immer die gleiche Wirkstoffmenge zu erhalten.

Außerdem hat diese Art der Formulierung mit feinkörnigen PVA-Qualitäten den Vorteil, dass die großen Oberflächen der feinen PVA-Partikel zu einer homogeneren Gelschichtbildung nach der Befeuchtung im Magen-DarmTrakt führt, was bei Einnahme der Tabletten durch den Patienten zu einer reproduzierbareren und ggf. auch verlängerten Diffusion des Wirkstoffs durch dieses Gel führt.

Durchführung

Zur Herstellung der erfindungsgemäßen Co-Mischungen werden feinkörnig gemahlene Polyvinylalkohole (PVAs) mit ausgewählten feinkörnigen mikrokristallinen Cellulosen (MCCs) intensiv vermischt und so in Co-Mischungen überführt, die als direkt verpressbare Tablettiermatrices hervorragend geeignet sind. Dieses ist besonders überraschend, da Abmischungen solcher PVAs mit anderen -per se auch sehr gut verpressbaren- direkt tablettierbaren Hilfsstoffen des Marktes diesen Direktverpressungseffekt mit den pulverförmigen PVAs nicht zeigen, insbesondere auch nicht mit beliebigen mikrokristallinen Cellulosen. Erst wenn feinkörnige PVAs mit besonders feinkörnigen mikrokristallinen Cellulosen kombiniert werden, ergeben sich direkt verpressbare Co-Mischungen.

Vorteilhafter Weise lassen sich mit diesen erfindungsgemäßen feinkörnigen Co-Mischungen an sich schlecht verpressbare Wirkstoffe in Formulierungen überführen, die ohne weitere Vorbereitungen sehr gut zu Tabletten verpresst werden können. Weiterhin kann mit den hergestellten Tabletten, die entsprechende Co-Mischungen als Wirkstoffträger enthalten, gezeigt werden, dass aus solchermaßen erzeugten Tabletten der Wirkstoff über sehr lange Zeit kontrolliert freigesetzt werden kann. Die entsprechenden wirkstoffhaltigen Tabletten zeigen verzögerte Wirkstofffreisetzungen von mindestens 2 Stunden, bevorzugt von über mindestens 6 Stunden, besonders bevorzugte von mindestens 8 Stunden, insbesondere bevorzugt von mindestens 10 Stunden, und ganz besonders bevorzugt Wirkstofffreisetzungen von bis zu 12 Stunden, abhängig vom eingesetzten Wirkstoff und vom Mischungsverhältnis der feinkörnigen Polyvinylalkohole mit den mikrokristallinen Cellulosen.

Da im Zusammenhang mit der Herstellung von Tablettenformulierungen der Begriff "direkt verpressbar" nicht verbindlich definiert ist wird in der vorliegenden Beschreibung das Pressverhalten eines als sehr gut verpressbaren Mannits des Marktes (Parteck® M 200 (Mannitol), geeignet für die Verwendung als Excipient EMPROVE® exp Ph. Eur, BP, JP, USP, E 421, Artikel Nr. 1.00419, Merck KGaA, Darmstadt, Deutschland) als Maßstab zum Vergleich verwendet. Ziel ist es, mit den direkt verpressbaren Co-Mischungen, die feinkörnigen PVAs in Kombination mit feinkörnigen mikrokristallinen Cellulosen in größerer Menge enthalten, insbesondere hinsichtlich ihrer Kompressibiliät dem Verhalten des Parteck® M 200 möglichst nahe zu kommen.

Durch die durchgeführten Versuche wurde gefunden, dass wirkstoffhaltige Tabletten, die eine erfindungsgemäße Zusammensetzung in Form einer Co-Mischung in einer Menge von 1-99 Gew.-%, bevorzugt in einer Menge von 5 - 95 Gew.-%, ganz besonders bevorzugt in einer Menge von 10-90 Gew.-% bezogen auf das Gesamtgewicht der Tablette enthalten, die gewünschte, besonders gute Kompressibilität aufweisen. Vorteilhafter Weise können mit solchen Zusammensetzungen wie gewünscht bereits bei Einsatz von niedrigen Presskräften Tabletten mit besonders hohe Tablettenhärten erhalten werden, die im Herstellungsprozess überraschend geringe Ausstoßkräfte erfordern. Bereits bei Einsatz einer Presskraft von 20 kN werden Tabletten mit einer Tablettenhärte von bis zu 462 N erhalten, die nur eine Ausstoßkraft von weniger als 60 N erfordern. Darüber hinaus weisen diese Tabletten nur geringe Friabilitäten auf, wie durch geeignete Versuche gezeigt werden kann.

Durch die vorliegende Erfindung wird somit ein Verfahren zur Herstellung von direkt verpressbaren Zusammensetzungen mit verlängerter Wirkstofffreisetzung und besonders guter Kompressibilität zur Verfügung gestellt, wodurch eine Co-Mischung aus feinkörnigen mikrokristallinen Cellulosen (MCCs) und feinkörnigen Polyvinylalkoholen (PVAs) herstellt wird, worin Polyvinylalkohol zu einem feinkörnigen Pulver mit einer mittleren Partikelgröße $D_{v50}$ im Bereich von 50 bis 260 µm, einer Schüttdichte im Bereich von 0,55 bis 0,62 g/ml und einem Schüttwinkel im Bereich von 35 bis 38° gemahlen und durch ein 800 µm Sieb gesiebt wird, und das erhaltene Pulver mit feinkörnigen mikrokristallinen Cellulosen (MCCs) mit mittleren Korngrößen von $D_{v50} < 70 µm$, bevorzugt mit mittleren Korngrößen im Bereich von $D_{v50}$ 17 bis 67 µm, insbesondere im Bereich von $D_{v50}$ 17 µm - 20 µm, und mit Schüttdichten im Bereich von 0,20 bis 0,35 g/cm$^3$, bevorzugt im Bereich von 0,20 bis 0,31 g/cm$^3$, vermischt wird Auf diese Weise wird eine direkt verpressbare Co-Mischung erhalten, die je nach Wunsch mit unterschiedlichen Wirkstoffen versetzt und zu Tabletten mit verzögerter Wirkstofffreisetzung verpresst werden kann.

Die im Folgenden gegebenen Beispiele offenbaren Methoden und Bedingungen zur Herstellung erfindungsgemäßer PVA-MCC-Co-Mischungen. Für den Fachmann auf dem Gebiet ist es selbsterklärend, dass auch andere Methoden zum Vermahlen und zum Vermischen der Ausgangssubstanzen zur Verfügung stehen als hier beschrieben sind.

Aus den Beispielen ergeben sich die besonderen Vorteile dieser feinkörnigen PVA-MCC- Kombinationen im Vergleich zu den ungenügenden Kompressibilitäten die durch PVA-Kombinationen mit anderen - aber als besonders gut tablettierbaren geltenden - Trägerstoffen erhalten werden. Bei Abmischung einer erfindungsgemäßen feinkörnigen PVA-MCC-Matrix mit einem per se schlecht verpressbaren pulverförmigen Wirkstoff und Zugabe einer sehr geringen Menge Magnesiumstearat als Schmiermittel können durch einfache Direkttablettierung Tabletten ausreichender Härten mit geringem mechanischem Abrieb erhalten werden, welche problemlos zur weiteren Behandlung z.B. zur Abfüllung in Blisterverpackungen oder zur bruchfreien Entnahme aus diesen Durchdrückpackungen durch den Patienten zur Verfügung stehen. Entsprechende Wirkstoff-haltige Tabletten zeigen, dass eine verlängerte Wirkstofffreisetzung aus solchen PVA-MCC-Matrixtabletten über mehrere Stunden gewährleistet werden kann.

Liste der Abbildungen:

In den Abbildungen Fig. 1 bis 4 sind die Versuchsergebnisse zur Veranschaulichung graphisch dargestellt:

**Figur 1:** Presskraft-Tablettenhärte-Profil (aus Tabelle 1b)

**Figur 2:** Presskraft-Tablettenhärte-Profil (aus Tabelle 2b)

**Figur 3:** Presskraft-Tablettenhärte-Profil (aus Tabelle 3b)

**Figur 4:** Presskraft-Tablettenhärte-Profil (aus Tabelle 4b)

Beispiele

Die vorliegende Beschreibung ermöglicht es dem Fachmann die Erfindung umfassend anzuwenden. Auch ohne weitere Ausführungen wird daher davon ausgegangen, dass ein Fachmann die obige Beschreibung im weitesten Umfang nutzen kann.

Bei etwaigen Unklarheiten versteht es sich von selbst, die zitierten Veröffentlichungen und Patentliteratur heranzuziehen. Dementsprechend gelten diese Dokumente als Teil der Offenbarung der vorliegenden Beschreibung.

Zum besseren Verständnis und zur Verdeutlichung der Erfindung werden im Folgenden Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew.- bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten daher %-Angaben als Gew.- oder mol-%, mit Ausnahme von Verhältnissen, die in Volumenangaben wiedergegeben sind.

Die in den Beispielen und der Beschreibung sowie in den Ansprüchen gegebenen Temperaturen gelten in °C.

Die Bedingungen zur Herstellung der speziellen erfindungsgemäßen PVA-MCC-Kombination ergeben sich aus den verschiedenen Beispielen. Ganz besonders geeignet sind die MCC-Typen Avicel PH105 (Beispiele A1-A4) und Avicel PH101 (Beispiele C1-C4) der Firma FMC Biopolymer sowie die Type Vivapur 101 (Beispiele B1-B4) der Firma JRS Pharma. Mit diesen Materialien werden die härtesten Tabletten erhalten bei Einsatz von vergleichbaren Presskräften, d.h. diese speziellen Kombinationen zeigen das beste "Dilution"-Potential.

**Charakterisierung der verwendeten Materialien**

**1. Verwendete PVA-Typen und ihre Eigenschaften:**

**1.1 Rohstoffe zur Mahlung**

1.1.1. PVA 4-88: Polyvinylalkohol 4-88, geeignet für die Verwendung als Excipient EMPROVE® exp Ph. Eur., USP, JPE, Artikel Nr. 1.41350, Merck KGaA, Darmstadt, Deutschland

1.1.2. PVA 18-88: Polyvinylalkohol 18-88, geeignet für die Verwendung als Excipient EMPROVE® exp Ph. Eur., USP, JPE, Artikel Nr. 1.41355, Merck KGaA, Darmstadt, Deutschland

1.1.3. PVA 26-88: Polyvinylalkohol 26-88, geeignet für die Verwendung als Excipient EMPROVE® exp Ph. Eur., USP, JPE, Artikel Nr. 1.41352, Merck KGaA, Darmstadt, Deutschland

1.1.4. PVA 40-88: Polyvinylalkohol 40-88, geeignet für die Verwendung als Excipient EMPROVE® exp Ph. Eur., USP, JPE, Artikel Nr. 1.41353, Merck KGaA, Darmstadt, Deutschland

1.1.5. PVA 28-99: Polyvinylalkohol 28-99, geeignet für die Verwendung als Excipient EMPROVE® exp JPE, Artikel Nr. 1.41356, Merck KGaA, Darmstadt, Deutschland

Diese PVA-Typen liegen als grobkörnige - mehrere Millimeter große - Partikel vor die in dieser Form nicht als eine direkt verpressbare Tablettiermatrix einsetzbar sind.

Die großen Partikel erlauben keine reproduzierbare Befüllung der Stempelmatrizen und somit auch kein konstantes Tablettengewicht bei hohen Rotationsgeschwindigkeiten der (Rundlauf)-Tablettiermaschinen. Außerdem können nur feinkörnige PVAs eine homogene Verteilung des Wirkstoffes ohne das Auftreten von Entmischungseffekten in den Tabletten gewährleisten. Dies ist für die Sicherstellung der Einzeldosiergenauigkeit des Wirkstoffes (content uniformity) in jeder produzierten Tablette unbedingt notwendig. Zusätzlich kann nur durch ein feinkörniges PVA auch die für die reproduzierbare Retardierung notwendige homogene Gelbildung im gesamten Tablettenkörper gewährleistet werden.

Aus diesen Gründen müssen die o.g. grobkörnigen PVA-Typen vor ihrer Verwendung als direkt verpressbare Retardierungsmatrices zerkleinert d.h. gemahlen werden,

**1.2 Gemahlene PVA-Typen**

1.2.1. Gemahlenes PVA 4-88, aus Polyvinylalkohol 4-88 Artikel Nr. 1.41350

1.2.2. Gemahlenes PVA 18-88, aus Polyvinylalkohol 18-88 Artikel Nr. 1.41355

1.2.3. Gemahlenes PVA 26-88, aus Polyvinylalkohol 26-88 Artikel Nr. 1.41352

1.2.4. Gemahlenes PVA 40-88, aus Polyvinylalkohol 40-88 Artikel Nr. 1.41353

1.2.5. Gemahlenes PVA 28-99, aus Polyvinylalkohol 28-99 Artikel Nr. 1.41356

Vermahlung:

Die Vermahlung der PVA-Typen erfolgt auf einer Aeroplex Spiralstrahlmühle Typ 200 AS der Firma Hosokawa Alpine, Augsburg, Deutschland unter flüssigem Stickstoff als Kaltvermahlung von 0°C bis minus 30°C,
Die resultierenden Produkteigenschaften der gemahlenen PVA-Typen insbes. die Pulverkennzahlen wie Schüttdichte, Stampfdichte, Schüttwinkel, BET-Oberfäche, BET-Porenvolumen sowie die Partikelgrößenverteilungen ergeben sich aus den nachfolgenden Tabellen:

Schüttdichte, Stampfdichte, Schüttwinkel, BET-Oberfläche, BET-Porenvolumen:

(Details zu den Messverfahren siehe unter Methoden)

| Muster | Schüttdichte (g/ml) | Stampfdichte (g/ml) | Schüttwinkel (°) | BET-Oberfläche ($m^2$/g) | BET-Porenvolumen ($cm^3$/g) |
|---|---|---|---|---|---|
| PVA 4-88* | 0,61 | 0,82 | 35,1 | 0,1308 | 0,0008 |
| PVA 18-88* | 0,57 | 0,76 | 35,5 | 0,1831 | 0,0011 |
| PVA 26-88* | 0,56 | 0,74 | 35,5 | 0,2045 | 0,0013 |
| PVA 40-88* | 0,59 | 0,77 | 36,9 | 0,1123 | 0,0009 |
| PVA 28-99* | 0,58 | 0,76 | 37,7 | 0,2210 | 0,0016 |
| * vermahlenes PVA | | | | | |

Partikelverteilung bestimmt über Laserbeugung mit Trockendispergierung (1 bar Gegendruck):

Angaben in $\mu$m (Details zum Messverfahren siehe unter Methoden)

| Muster | Dv5 | Dv10 | Dv20 | Dv25 | Dv30 | Dv50 | Dv75 | Dv90 |
|---|---|---|---|---|---|---|---|---|
| PVA 4-88* | 21,36 | 33,93 | 60,39 | 75,25 | 91,61 | 177,74 | 380,57 | 790,37 |
| PVA 18-88* | 29,67 | 44,93 | 73,95 | 89,11 | 105,22 | 185,49 | 375,88 | 755,84 |
| PVA 26-88* | 27,76 | 42,32 | 73,01 | 90,14 | 108,67 | 198,51 | 382,65 | 676,96 |
| PVA 40-88* | 31,84 | 50,64 | 89,13 | 109,77 | 131,45 | 230,52 | 413,71 | 634,59 |
| PVA 28-99* | 24,87 | 39,81 | 72,81 | 90,72 | 109,31 | 191,42 | 343,54 | 561,23 |
| * vermahlenes PVA | | | | | | | | |

Partikelverteilung bestimmt über Laserbeugung mit Trockendispergierung (2 bar Gegendruck):

Angaben in $\mu$m (Details zum Messverfahren siehe unter Methoden)

| Muster | Dv5 | Dv10 | Dv20 | Dv25 | Dv30 | Dv50 | Dv75 | Dv90 |
|---|---|---|---|---|---|---|---|---|
| PVA 4-88* | 19,09 | 30,21 | 52,69 | 64,83 | 77,87 | 143,83 | 279,64 | 451,94 |
| PVA 18-88* | 26,90 | 40,38 | 65,3 | 78,08 | 91,55 | 159,10 | 321,46 | 607,64 |
| PVA 26-88* | 24,59 | 36,93 | 61,67 | 75,05 | 89,33 | 157,79 | 286,17 | 434,23 |
| PVA 40-88* | 31,03 | 49,47 | 88,54 | 110,06 | 132,79 | 235,87 | 430,35 | 686,1 |
| PVA 28-99* | 24,27 | 39,63 | 74,31 | 93,13 | 112,51 | 196,45 | 350,21 | 570,12 |
| * vermahlenes PVA | | | | | | | | |

Partikelverteilung bestimmt über Laserbeugung mit Trockendispergierung (3 bar Gegendruck):

Angaben in $\mu$m (Details zum Messverfahren siehe unter Methoden)

| Muster | Dv5 | Dv10 | Dv20 | Dv25 | Dv30 | Dv50 | Dv75 | Dv90 |
|---|---|---|---|---|---|---|---|---|
| PVA 4-88* | 18,35 | 29,27 | 51,25 | 63,09 | 75,77 | 139,46 | 269,8 | 425,62 |
| PVA 18-88* | 24,55 | 36,60 | 57,91 | 68,48 | 79,45 | 132,37 | 246,56 | 393,59 |
| PVA 26-88* | 25,17 | 38,18 | 64,35 | 78,47 | 93,57 | 167,41 | 317,16 | 514,18 |
| PVA 40-88* | 32,81 | 53,33 | 96,27 | 119,61 | 144,21 | 256,31 | 463,67 | 717,76 |
| PVA 28-99* | 22,33 | 35,92 | 65,94 | 82,31 | 99,37 | 174,84 | 305,5 | 454,03 |
| * vermahlenes PVA | | | | | | | | |

Partikelverteilung bestimmt über Laserbeugung mit Nassdispergierung (in dünnflüssigem Silikonöl):

Angaben in $\mu$m (Details zum Messverfahren siehe unter Methoden)

| Muster | Dv5 | Dv10 | Dv20 | Dv25 | Dv30 | Dv50 | Dv75 | Dv90 |
|---|---|---|---|---|---|---|---|---|
| PVA 4-88* | 10,03 | 20,1 | 38,02 | 47,82 | 58,31 | 110,91 | 231,64 | 390,95 |
| PVA 18-88* | 17,19 | 30,25 | 50,06 | 59,22 | 68,47 | 111,89 | 212,70 | 357,70 |
| PVA 26-88* | 15,42 | 26,76 | 45,50 | 54,83 | 64,47 | 110,50 | 212,91 | 353,68 |
| PVA 40-88* | 20,41 | 34,80 | 60,35 | 73,32 | 86,96 | 154,96 | 299,57 | 490,08 |
| PVA 28-99* | 14,68 | 25,96 | 47,49 | 58,88 | 70,80 | 127,68 | 240,70 | 376,70 |
| * vermahlenes PVA | | | | | | | | |

Partikelverteilung bestimmt über Turmsiebung:

Angaben in Gewichtsprozent (Details zum Messverfahren siehe unter Methoden)

| Muster | <32 µm | 32-50 µm | 50-75 µm | 75-100 µm | 100-150 µm | 150-200 µm | 200-250 µm |
|---|---|---|---|---|---|---|---|
| PVA 4-88* | 3,3 | 7,9 | 12,6 | 12,2 | 19,6 | 12,9 | 10,5 |
| PVA 18-88* | 0,5 | 8,1 | 12,8 | 13,6 | 20,4 | 15,0 | 9,4 |
| PVA 26-88* | 5,3 | 8,4 | 12,3 | 13,6 | 21,8 | 13,1 | 9,0 |
| PVA 40-88 | 2,6 | 5,5 | 8,1 | 8,8 | 17,8 | 14,0 | 10,7 |
| PVA 28-99* | 5,0 | 7,1 | 9,1 | 9,8 | 20,4 | 13,2 | 11,7 |

| Muster | 250-300 µm | 300-355 µm | 355-400 µm | 400-500 µm | 500-600 µm | 600-710 µm | >710 µm |
|---|---|---|---|---|---|---|---|
| PVA 4-88* | 6,5 | 4,5 | 2,8 | 3,5 | 2,0 | 0,9 | 0,8 |
| PVA 18-88* | 5,8 | 4,2 | 2,6 | 3,5 | 2,1 | 1,0 | 1,0 |
| PVA 26-88* | 5,0 | 3,7 | 2,2 | 2,7 | 1,8 | 0,6 | 0,5 |
| PVA 40-88 | 7,5 | 6,6 | 3,9 | 5,9 | 4,1 | 1,9 | 2,6 |
| PVA 28-99* | 7,9 | 5,3 | 3,2 | 3,7 | 2,0 | 0,8 | 0,8 |

\* vermahlenes PVA

**2. Mikrokristalline Cellulosen (MCCs) zur Herstellung der Abmischungen mit Polyvinylalkoholen (vermahlen)**

2.1 Avicel® PH 101, mikrokristalline Cellulose, Ph. Eur., NF, JP, FMC BioPolymer, USA

2.2 Avicel® PH 102, mikrokristalline Cellulose, Ph. Eur., NF, JP, FMC BioPolymer, USA

2.3 Avicel® PH 102 SCG, mikrokristalline Cellulose, Ph. Eur., NF, JP, FMC BioPolymer, USA

2.4 Avicel® PH 105, mikrokristalline Cellulose, Ph. Eur., NF, JP, FMC BioPolymer, USA

2.5 Vivapur® Type 12, mikrokristalline Cellulose, Ph. Eur., NF, JP, JRS Pharma, Rosenberg, Deutschland

2.6 Vivapur® Type 101, mikrokristalline Cellulose, Ph. Eur., NF, JP, JRS Pharma, Rosenberg, Deutschland

2.7 Vivapur® Type 102 Premium, mikrokristalline Cellulose, Ph. Eur., NF, JP, JRS Pharma, Rosenberg, Deutschland

2.8 Vivapur® Type 200, mikrokristalline Cellulose, Ph. Eur., NF, JP, JRS Pharma, Rosenberg, Deutschland

2.9 Emcocel® 90 M, mikrokristalline Cellulose, Ph. Eur., NF, JP, JRS Pharma, , Rosenberg, Deutschland

2.10 Emcocel® LP 200, mikrokristalline Cellulose, Ph. Eur., NF, JP, JRS Pharma, , Rosenberg, Deutschland

2.11 Comprecel® M 302, mikrokristalline Cellulose, Ph. Eur., NF, JP, BP, USP, Mingtai Chemical Co. Ltd., Taiwan

Partikelverteilung bestimmt über Laserbeugung mit Trockendispergierung (1 bar Gegendruck):

Angaben in µm (Details zum Messverfahren siehe unter Methoden)

| Muster | Dv10 | Dv20 | Dv25 | Dv30 | Dv50 | Dv75 | Dv90 |
|---|---|---|---|---|---|---|---|
| Avicel® PH 101 | 22,59 | 33,09 | 37,77 | 42,36 | 61,82 | 98,62 | 161,34 |
| Avicel® PH 102 | 28,27 | 46,75 | 56,59 | 66,56 | 107,27 | 170,38 | 235,70 |
| Avicel® PH 102 SCG | 48,99 | 90,03 | 106,32 | 120,84 | 173,66 | 251,80 | 331,64 |
| Avicel® PH 105 | 6,80 | 10,21 | 11,61 | 12,94 | 18,50 | 28,35 | 40,38 |

(fortgesetzt)

| Muster | Dv10 | Dv20 | Dv25 | Dv30 | Dv50 | Dv75 | Dv90 |
|---|---|---|---|---|---|---|---|
| Vivapur® 12 | 42,55 | 75,61 | 92,59 | 108,97 | 171,37 | 264,07 | 358,09 |
| Vivapur® 101 | 20,66 | 30,70 | 35,97 | 41,53 | 66,58 | 108,89 | 155,53 |
| Vivapur® 102 | 31,56 | 53,04 | 66,00 | 79,89 | 135,87 | 215,53 | 293,94 |
| Vivapur® 200 | 49,25 | 97,09 | 125,64 | 152,47 | 245,21 | 375,17 | 507,15 |
| Emcocel® 90 M | 41,28 | 63,99 | 73,89 | 83,41 | 121,96 | 185,25 | 253,79 |
| Emcocel® LP 200 | 68,47 | 113,69 | 129,77 | 144,39 | 199,67 | 285,27 | 376,22 |
| Comprecel® M 302 | 30,07 | 55,56 | 66,85 | 77,23 | 116,30 | 176,60 | 240,36 |

Partikelverteilung bestimmt über Laserbeugung mit Trockendispergierung (2 bar Gegendruck):

Angaben in $\mu$m (Details zum Messverfahren siehe unter Methoden)

| Muster | Dv10 | Dv20 | Dv25 | Dv30 | Dv50 | Dv75 | Dv90 |
|---|---|---|---|---|---|---|---|
| Avicel® PH 101 | 19,43 | 28,55 | 32,60 | 36,53 | 52,81 | 80,77 | 114,13 |
| Avicel® PH 102 | 28,40 | 47,32 | 57,45 | 67,69 | 108,91 | 171,94 | 236,64 |
| Avicel® PH 102 SCG | 48,32 | 84,95 | 100,38 | 114,43 | 166,33 | 243,47 | 321,96 |
| Avicel® PH 105 | 6,39 | 9,81 | 11,19 | 12,52 | 18,03 | 27,77 | 39,70 |
| Vivapur® 12 | 35,98 | 62,68 | 77,81 | 93,33 | 155,79 | 249,72 | 345.23 |
| Vivapur® 101 | 19,61 | 29,42 | 34,61 | 40,15 | 66,06 | 113,18 | 176,82 |
| Vivapur® 102 | 27,55 | 45,97 | 57,41 | 70,40 | 127,29 | 208,92 | 288,93 |
| Vivapur® 200 | 44,08 | 86,21 | 113,63 | 140,90 | 235,62 | 365,86 | 497,34 |
| Emcocel® 90 M | 37,39 | 58,75 | 68,08 | 77,03 | 113,34 | 173,41 | 239,37 |
| Emcocel® LP 200 | 75,97 | 121,31 | 137,44 | 152,19 | 208,23 | 294,84 | 385,17 |
| Comprecel® M 302 | 33,33 | 62,38 | 74,56 | 85,63 | 127,04 | 190,77 | 257,84 |

Partikelverteilung bestimmt über Laserbeugung mit Trockendispergierung (3 bar Gegendruck):

Angaben in $\mu$m (Details zum Messverfahren siehe unter Methoden)

| Muster | Dv10 | Dv20 | Dv25 | Dv30 | Dv50 | Dv75 | Dv90 |
|---|---|---|---|---|---|---|---|
| Avicel® PH 101 | 18,03 | 26,91 | 30,91 | 34,81 | 51,16 | 80,11 | 117,89 |
| Avicel® PH 102 | 24,28 | 40,18 | 49,21 | 58,86 | 100,25 | 164,22 | 229,95 |
| Avicel® PH 102 SCG | 42,19 | 77,05 | 92,59 | 106,73 | 158,55 | 234,98 | 312,72 |
| Avicel® PH 105 | 6,10 | 9,50 | 10,88 | 12,20 | 17,67 | 27,29 | 38,96 |
| Vivapur® 12 | 31,65 | 54,13 | 67,50 | 81,98 | 144,53 | 240,48 | 338,01 |
| Vivapur® 101 | 17,23 | 25,91 | 30,40 | 35,18 | 58,17 | 99,16 | 143,94 |
| Vivapur® 102 | 23,61 | 38,84 | 48,19 | 59,22 | 114,76 | 198,37 | 278,99 |
| Vivapur® 200 | 38,43 | 73,36 | 97,85 | 124,94 | 223,50 | 356,46 | 490,73 |

(fortgesetzt)

| Muster | Dv10 | Dv20 | Dv25 | Dv30 | Dv50 | Dv75 | Dv90 |
|---|---|---|---|---|---|---|---|
| Emcocel® 90 M | 34,07 | 55,25 | 64,57 | 73,49 | 109,27 | 167,95 | 232,86 |
| Emcocel® LP 200 | 61,18 | 104,76 | 120,78 | 135,31 | 189,83 | 272,98 | 358,76 |
| Comprecel® M 302 | 29,22 | 54,80 | 66,28 | 76,75 | 115,86 | 175,96 | 239,63 |
| * vermahlenes PVA | | | | | | | |

Partikelverteilung bestimmt über Laserbeugung mit Nassdispergierung (in dünnflüssigem Silikonöl):

Angaben in $\mu$m (Details zum Messverfahren siehe unter Methoden)

| Muster | Dv10 | Dv20 | Dv25 | Dv30 | Dv50 | Dv75 | Dv90 |
|---|---|---|---|---|---|---|---|
| Avicel® PH 101 | 20,66 | 32,85 | 38,18 | 43,31 | 63,99 | 98,56 | 140,53 |
| Avicel® PH 102 | 26,92 | 46,05 | 55,55 | 64,77 | 101,48 | 161,28 | 227,07 |
| Avicel® PH 102 SCG | 38,64 | 69,23 | 83,63 | 97,33 | 150,39 | 231,75 | 316,41 |
| Avicel® PH 105 | 5,21 | 9,07 | 10,51 | 11,84 | 17,11 | 26,17 | 37,37 |
| Vivapur® 12 | 31,45 | 55,34 | 67,86 | 80,26 | 132,04 | 219,78 | 316,04 |
| Vivapur® 101 | 17,51 | 26,83 | 31,53 | 36,51 | 59,93 | 99,84 | 144,07 |
| Vivapur® 102 | 28,28 | 47,27 | 58,07 | 69,46 | 119,03 | 200,35 | 285,42 |
| Vivapur® 200 | 33,53 | 59,12 | 74,18 | 90,77 | 171,42 | 302,56 | 434,89 |
| Emcocel® 90 M | 35,68 | 58,96 | 68,77 | 78,12 | 116,55 | 183,76 | 261,39 |
| Emcocel® LP 200 | 60,38 | 105,52 | 122,18 | 137,35 | 194,75 | 283,57 | 377,02 |
| Comprecel® M 302 | 27,02 | 52,05 | 63,61 | 74,24 | 114,48 | 178,54 | 248,78 |

## 2. Übrige Materialien

Da der Begriff "direkt verpressbar" nicht verbindlich definiert ist wird das Pressverhalten eines sehr gut verpressbaren Mannits des Marktes als Maßstab eingesetzt:

Parteck® M 200 (Mannitol), geeignet für die Verwendung als Excipient EMPROVE® exp Ph. Eur., BP, JP, USP, E 421, Artikel Nr. 1.00419, Merck KGaA, Darmstadt, Deutschland

Ziel ist es mit den direkt verpressbaren PVAs insbes. hinsichtlich ihrer Kompressibiliät dem Verhalten des Parteck® M 200 möglichst nahe zu kommen.

**Geräte/Verfahren zur Charakterisierung der Stoffeigenschaften**

1. Schüttdichte: gemäß DIN EN ISO 60: 1999 (Deutsche Fassung) - Angabe in "g/ml"

2. Stampfdichte: gemäß DIN EN ISO 787-11: 1995 (Deutsche Fassung) - Angabe in "g/ml"

3. Schüttwinkel: gemäß DIN ISO 4324: 1983 (Deutsche Fassung) - Angabe in "Grad"

4. Oberfläche bestimmt gemäß BET: Auswertung und Durchführung gemäß Literatur "BET Surface Area by Nitrogen Absorption" von S.Brunauer et al. (Journal of American Chemical Society, 60, 9, 1983) Gerät: ASAP 2420 Fa.

Micromeritics Instrument Corporation (USA); Stickstoff; Einwaage: ca. 3,0000 g; Ausheizung: 50°C (5 h); Heizrate 3K/min; Angabe des arithmetischen Mittelwertes aus drei Bestimmungen

5. Partikelgrößenbestimmung über Laserbeugung mit Trockendispergierung: Mastersizer 2000 mit Dispergiereinheit Scirocco 2000 (Fa. Malvern Instruments Ltd. UK), Bestimmungen bei 1, 2 und 3 bar Gegendruck; Auswertung Fraunhofer; Dispersant RI: 1.000, Obscuration Limits: 0.1-10.0%, Tray Type: General Purpose, Background Time: 7500 msec, Measurement Time: 7500 msec, Durchführung gemäß ISO 13320-1 sowie den Angaben des technischen Handbuchs und den Spezifikationen des Geräteherstellers; Angaben in Vol%

6. Partikelgrößenbestimmung über Laserbeugung mit Naßdispergierung: Mastersizer 2000 mit Naßdispergiereinheit Hydro 2000SM (Fa. Malvern Instruments Ltd. UK); Dispersionsmedium Silikonöl dünnflüssig (Hersteller: Evonic Goldschmidt GmbH, Deutschland; Bezeichnung des Herstellers: Tegiloxan3, Artikelnummer des Herstellers: 9000305); Dispersant RI: 1.403; Stirrer Speed: 2500 rpm; Tray Type: General Purpose; Background Time: 7500 msec; Measurement Time: 7500 msec; Obscuration Limits: 7.0-13.0%; Durchführung gemäß ISO 13320-1 sowie den Angaben des technischen Handbuchs und den Spezifikationen des Geräteherstellers; Angaben in Vol%. Durchführung: Die Suspensionszelle wird mit dem dünnflüssigen Silikonöl gefüllt, portionsweise die Probe bis zum Erreichen des angestrebten Obscurationbereichs (7,0-13,0%) zugegeben und nach 2 Minuten Wartezeit die Messung gestartet.

7. Partikelgrößenbestimmung durch Trockensiebung über einen Siebturm: Retsch AS 200 control, Fa.Retsch (Deutschland); Substanzmenge: ca. 110,00 g; Siebzeit: 30 Minuten; Amplitudenintensität: 1mm; Intervall: 5 Sekunden; Analysensiebe mit Metalldrahtgewebe gemäß DIN ISO 3310; Siebweiten (in $\mu$m): 710, 600, 500, 400, 355, 300, 250, 200, 150, 100, 75, 50, 32; Angabe der Mengenverteilung pro Siebfraktion in den Tabellen als "Gew.-% der Einwaage"

8. Die Tablettierungsprüfungen erfolgen folgendermaßen:
Die Mischungen gemäß der im Versuchsteil angegebenen Zusammensetzungen werden 5 Minuten in einem verschlossenen Edelstahlbehälter (Fassungsvolumen: ca. 2 l, Höhe: ca. 19,5 cm, Durchmesser: ca. 12 cm Außenmaß) auf einem Labor-Taumelmischer (Turbula T2A, Fa.Willy A. Bachofen, Schweiz) gemischt.

Als Magnesiumstearat wird Parteck LUB MST (Magnesiumstearat pflanzlich) EMPROVE exp Ph. Eur., BP, JP, NF, FCC Artikel Nr. 1.00663 (Merck KGaA, Deutschland) eingesetzt welches über ein 250 $\mu$m Sieb abgelegt wurde.
Die Verpressung zu 500 mg Tabletten (11 mm Stempel, rund, flach, mit Facette) erfolgt auf einer instrumentierten Excenter-Tablettiermaschine Korsch EK 0-DMS (Fa. Korsch, Deutschland) mit dem Auswertesystem Catman 5.0 (Fa. Hottinger Baldwin Messtechnik - HBM, Deutschland).
Je getesteter Presskraft (Soll-Einstellungen: ~5, ~10, ~20 und ~30 kN; die effektiv gemessenen Ist-Werte sind in den Beispielen angegeben) werden mindestens 100 Tabletten zur Auswertung der Pressdaten und Bestimmung der galenischen Kennzahlen hergestellt.
Tablettenhärten, Durchmesser und Höhen: Erweka Multicheck 5.1 (Fa. Erweka, Deutschland); Durchschnittsdaten (arithmetische Mittelwerte) aus jeweils 20 Tablettenmessungen pro Presskraft. Die Messungen erfolgen einen Tag nach der Tablettenherstellung.
Tablettenabrieb: Friabilitätsprüfgerät TA420 (Fa. Erweka, Deutschland); Geräteparameter und Ausführung der Messungen gemäß Ph. Eur. 7.Ausgabe "Friabilität von nicht überzogenen Tabletten". Die Messungen erfolgen einen Tag nach der Tablettenherstellung.
Tablettenmasse: Durchschnittswert (arithmetischer Mittelwert) aus der Wägung von 20 Tabletten je Presskraft: Multicheck 5.1 (Fa. Erweka, Deutschland) mit Waage Sartorius CPA 64 (Fa. Sartorius, Deutschland). Die Messungen erfolgen einen Tag nach der Tablettenherstellung.

**Versuchsergebnisse**

Es wurde durch die Versuche gefunden, dass insbesondere nur die Co-Mischungen mit drei speziellen mikrokristallinen Cellulosen(MCCs) zu einer guten Verpressbarkeit führen.
Durch die Versuche wurde auch gefunden, dass offensichtlich nicht alle im Handel erhältlichen MCC-Typen eine Verbesserung der Kompressibilität in den Co-Mischungen mit gemahlenen PVAs zeigen.
Da der Begriff "direkt verpreßbar" nicht verbindlich definiert ist wird das Pressverhalten eines als sehr gut verpressbaren Mannits des Marktes (Parteck® M 200 (Mannitol)), geeignet für die Verwendung als Excipient EMPROVE® exp Ph. Eur., BP, JP, USP, E 421, Katalog Nr, 100419, Merck KGaA, Darmstadt, Deutschland) als Maßstab gesetzt. Ziel ist es mit den direkt verpressbaren PVAs (als Co-Mischungen) insbesondere hinsichtlich ihrer Kompressibilität dem Verhalten des Parteck® M200 möglichst nahe zu kommen.

Es wurde durch die Versuche gefunden, dass Co-Mischungen auf Basis von feinkörnig gemahlenen Polyvinylalkoholen mit den feinkörnigen mikrokristallinen Cellulosen, wie beispielsweise mit den im Handel erhältlichen Produkten Avicel PH 105 (Beispiele A1-A4), Vivapur 101 (Beispiele B1-B4) und Avicel PH101 (Beispiele C1-C4) eine ganz besonders gute Kompressibilität aufweisen. Diese Kompressibiliät ist gleichwertig oder sogar noch deutlich besser als diejenige des als besonders gut direkt verpressbar geltenden Parteck® M200.

Diese speziellen PVA-MCC-Co-Mischungen sind damit als Matrices zur Formulierung von Retardtabletten in Kombination mit an sich schlecht verpressbaren Wirkstoffen in der Direkttablettierung besonders gut geeignet.

**Voraehensweise:**

1a. Herstellung der Abmischungen bestehend aus den verschiedenen mikrokristallinene Cellulosen des Marktes mit der gemahlenen PVA-Type 4-88

1b. Verpressung dieser Abmischungen (mit Zusatz von 0,25 Gew.-% Parteck® LUB MST) und Tablettencharakterisierung hinsichtlich der Parameter Tablettenhärte, Tablettenmasse, Tablettenhöhe, Tablettenabrieb sowie notwendiger Ausstoßkraft

2a. Herstellung der Abmischungen bestehend aus den verschiedenen mikrokristallinene Cellulosen des Marktes mit der gemahlenen PVA-Type 18-88

2b. Verpressung dieser Abmischungen (mit Zusatz von 0,25 Gew.-% Parteck® LUB MST) und Tablettencharakterisierung hinsichtlich der Parameter Tablettenhärte, Tablettenmasse, Tablettenhöhe, Tablettenabrieb sowie notwendiger Ausstoßkraft

3a. Herstellung der Abmischungen bestehend aus den verschiedenen mikrokristallinene Cellulosen des Marktes mit der gemahlenen PVA-Type 26-88

3b. Verpressung dieser Abmischungen (mit Zusatz von 0,25 Gew.-% Parteck® LUB MST) und Tablettencharakterisierung hinsichtlich der Parameter Tablettenhärte, Tablettenmasse, Tablettenhöhe, Tablettenabrieb sowie notwendiger Ausstoßkraft

4a. Herstellung der Abmischungen bestehend aus den verschiedenen mikrokristallinene Cellulosen des Marktes mit der gemahlenen PVA-Type 40-88

4b. Verpressung dieser Abmischungen (mit Zusatz von 0,25 Gew.-% Parteck® LUB MST) und Tablettencharakterisierung hinsichtlich der Parameter Tablettenhärte, Tablettenmasse, Tablettenhöhe, Tablettenabrieb sowie notwendiger Ausstoßkraft

**A) Versuchsergebnisse:**

**1a. Herstellung der Abmischungen der direkt verpressbaren Trägerstoffe mit der gemahlenen PVA-Type 4-88**

Allgemeine Beschreibung: gemahlenes PVA 4-88 wird zur Entfernung von eventuellen Grobanteilen und Agglomeraten durch ein 800 $\mu$m Handsieb abgelegt. 300 g dieser gesiebten Ware werden in ein 2l Turbulamischgefäß eingewogen, 300 g der entsprechenden mikrokristallinen Cellulose aus Tabelle 1a hinzugegeben und 5 min. in einem Turbulamischer T2A gemischt.

**Tabelle 1a: Zusammensetzung der Co-Mischungen gemahlenes PVA 4-88 mit mikrokristallinen Cellulosen**

| Zusammensetzung | 50 Gew.-% PVA | 50 Gew.-% MCC |
|---|---|---|
| Beispiel A1 | PVA 4-88* | Avicel® PH 105 |
| Beispiel B1 | PVA 4-88* | Vivapur® 101 |
| Beispiel C1 | PVA 4-88* | Avicel® PH 101 |

(fortgesetzt)

| Zusammensetzung | 50 Gew.-% PVA | 50 Gew.-% MCC |
|---|---|---|
| Vergleich D1 | PVA 4-88* | Vivapur® 12 |
| Vergleich E1 | PVA 4-88* | Vivapur® 102 Premium |
| Vergleich F1 | PVA 4-88* | Vivapur® 200 |
| Vergleich G1 | PVA 4-88* | Emcocel® LP200 |
| *: vermahlenes PVA | | |

## 1b. Verpressung dieser Abmischungen und Tablettencharakterisierung

Allg. Beschreibung: je 498,75 g der oben hergestellten Co-Mischungen aus den Beispielen A1-C1 bzw. den Vergleichen D1-G1 werden in einem Turbulamischgefäß mit 1,25g Magnesiumstearat versetzt, nochmals 5 min. in einem Turbulamischer T2A gemischt und an einer Exzenterpresse Korsch EK 0-DMS tablettiert.

Als Vergleich dient Parteck® M200 abgemischt mit 1% Parteck® LUB MST. Anmerkung: eine Verpressung des Parteck® M200 mit weniger Magnesiumstearat ist wegen der ansonst resultierenden sehr hohen Ausstoßkräfte nicht möglich.

### Tabelle 1b: Tablettierdaten der Co-Mischungen gemahlenes PVA 4-88 mit mikrokristallinen Cellulosen

Legende:

A: Presskraft [kN]          B: Tablettenhärte nach 1 Tag [N]
C: Tablettenmasse [mg]      D: Tablettenhöhe [mm]
E: Abrieb [%]              F: Ausstoßkraft(N)

| | A | | B | C | D | E | F |
|---|---|---|---|---|---|---|---|
| | Soll | Ist | | | | | |
| **Beispiel A1** | 5 | 4,9 | 102,7 | 498,6 | 5,4 | 0,24 | 103,3 |
| | 10 | 10,4 | 230,8 | 493,1 | 4,8 | 0 | 110,1 |
| | 20 | 19,5 | 439,4 | 486,6 | 4,4 | 0 | 70,4 |
| | 30 | 30,3 | 551,5 | 486,9 | 4,3 | 0 | 48,6 |
| **Beispiel B1** | 5 | 5,1 | 89,6 | 500,8 | 5,5 | 0,43 | 90,1 |
| | 10 | 9,5 | 192,7 | 500,4 | 4,9 | 0,16 | 94,6 |
| | 20 | 21,4 | 390,1 | 504,9 | 4,5 | 0,07 | 58,8 |
| | 30 | 29,5 | 447,6 | 504,3 | 4,4 | 0,07 | 51,2 |
| **Beispiel C1** | 5 | 4,9 | 77,9 | 495,1 | 5,6 | 0,69 | 96,8 |
| | 10 | 9,8 | 178,6 | 497,8 | 4,9 | 0,16 | 98,7 |
| | 20 | 21,1 | 340,5 | 501,6 | 4,5 | 0,06 | 61,7 |
| | 30 | 30,9 | 405,0 | 503,6 | 4,4 | 0,05 | 50,7 |
| **Vergleich D1** | 5 | 5,0 | 45,8 | 495,8 | 5,4 | 1,47 | 86,6 |
| | 10 | 10,6 | 107,0 | 500,5 | 4,9 | 0,27 | 97,5 |
| | 20 | 20,2 | 208,9 | 502,0 | 4,4 | 0,08 | 75,4 |
| | 30 | 30,7 | 250,8 | 502,4 | 4,4 | 0,07 | 66,4 |
| **Vergleich E1** | 5 | 5,1 | 65,2 | 501,3 | 5,5 | 0,55 | --- |
| | 10 | 9,6 | 140,0 | 504,8 | 4,9 | 0,19 | 95,9 |
| | 20 | 19,8 | 264,8 | 503,9 | 4,5 | 0,10 | 65,4 |
| | 30 | 30,8 | 321,2 | 504,7 | 4,4 | 0,06 | 56,3 |
| **Vergleich F1** | 5 | 5,1 | 33,7 | 497,8 | 5,5 | 4,72 | 75,4 |
| | 10 | 9,6 | 81,2 | 502,1 | 5,0 | 0,59 | 85,7 |
| | 20 | 19,1 | 160,4 | 503,2 | 4,6 | 0,19 | 62,4 |

(fortgesetzt)

| Legende: A: Presskraft [kN] C: Tablettenmasse [mg] E: Abrieb [%] | | B: Tablettenhärte nach 1 Tag [N] D: Tablettenhöhe [mm] F: Ausstoßkraft(N) | | | | | |
|---|---|---|---|---|---|---|---|
| | 30 | 30,3 | 188,8 | 502,2 | 4,5 | 0,14 | 53,9 |
| **Vergleich G1** | 5 | 5,0 | 22,4 | 493,4 | 5,7 | 47,34 | 82,0 |
| | 10 | 9,7 | 58,2 | 498,0 | 5,0 | 1,24 | 91,1 |
| | 20 | 20,0 | 121,6 | 500,4 | 4,6 | 0,25 | 63,8 |
| | 30 | 29,6 | 138,3 | 500,8 | 4,5 | 0,21 | 54,7 |
| **Parteck® M200** | 5 | 5,2 | 84,1 | 497,8 | 5,1 | 0,21 | 155,8 |
| | 10 | 10,7 | 196,5 | 500,6 | 4,6 | 0,17 | 306,0 |
| | 20 | 20,3 | 340,0 | 499,4 | 4,2 | 0,15 | 513,6 |
| | 30 | 30,0 | 396,7 | 498,3 | 4,0 | 0,16 | 647,6 |

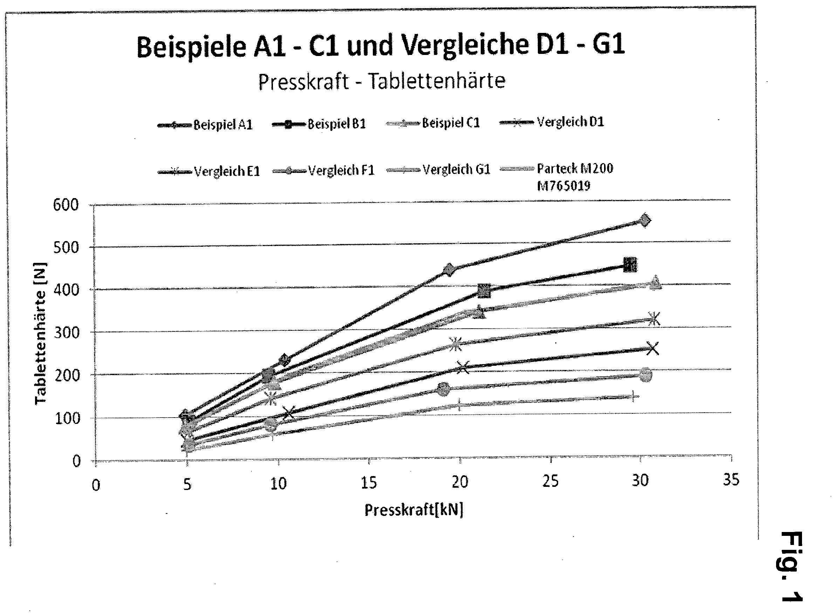

In der Figur 1 sind die stark unterschiedlichen Preßkraft-Tablettenhärte-Profile zur besseren Veranschaulichung graphisch dargestellt.

**2a. Herstellung der Abmischungen der direkt verpressbaren Trägerstoffe mit der gemahlenen PVA-Type 18-88**

Allgemeine Beschreibung: gemahlenes PVA 18-88 wird zur Entfernung von eventuellen Grobanteilen und Agglomeraten durch ein 800 μm Handsieb abgelegt. 300 g dieser gesiebten Ware werden in ein 2l Turbulamischgefäß eingewogen, 300 g der entsprechenden mikrokristallinen Cellulose aus Tabelle 2a hinzugegeben und 5 min. in einem Turbulamischer T2A gemischt.

**Tabelle 2a: Zusammensetzung der Co-Mischungen gemahlenes PVA 18-88 mit mikrokristallinen Cellulosen**

| Zusammensetzung | 50 Gew.-% PVA | 50 Gew.-% MCC |
|---|---|---|
| Beispiel A2 | PVA 18-88* | Avicel® PH 105 |
| Beispiel B2 | PVA 18-88* | Vivapur® 101 |
| Beispiel C2 | PVA 18-88* | Avicel® PH 101 |
| Vergleich D2 | PVA 18-88* | Vivapur® 12 |
| Vergleich E2 | PVA 18-88* | Vivapur® 102 Premium |
| Vergleich F2 | PVA 18-88* | Vivapur® 200 |
| Vergleich G2 | PVA 18-88* | Emcocel® LP200 |
| *: vermahlenes PVA | | |

**2b. Verpressung dieser Abmischungen und Tablettencharakterisierung**

Allgemeine Beschreibung:
je 498,75 g der oben hergestellten Co-Mischungen aus den Beispielen A2-C2 bzw. den Vergleichen D2-G2 werden in einem Turbulamischgefäß mit 1,25 g Magnesiumstearat versetzt, nochmals 5 min. in einem Turbulamischer T2A gemischt und an einer Exzenterpresse Korsch EK 0-DMS tablettiert.
Als Vergleich dient Parteck® M200 abgemischt mit 1% Parteck® LUB MST. Anmerkung: eine Verpressung des Parteck® M200 mit weniger Magnesiumstearat ist wegen der ansonst resultierenden sehr hohen Ausstoßkräfte nicht möglich.

**Tabelle 2b: Tablettierdaten der Co-Mischungen gemahlenes PVA 18-88 mit mikrokristallinen Cellulosen**

Legende:

A: Presskraft [kN]                      B: Tablettenhärte nach 1 Tag [N]
C: Tablettenmasse [mg]                D: Tablettenhöhe [mm]
E: Abrieb [%]                            F: Ausstoßkraft(N)

| | A | | B | c | D | E | F |
| --- | --- | --- | --- | --- | --- | --- | --- |
| | Soll | Ist | | | | | |
| **Beispiel A2** | 5 | 5,6 | 120,0 | 501,1 | 5,4 | 0,08 | 107,2 |
| | 10 | 10,3 | 239,1 | 501,9 | 4,9 | 0 | 108,8 |
| | 20 | 20,5 | 465,5 | 502,2 | 4,5 | 0 | 69,5 |
| | 30 | 31,1 | 591,0 | 497,2 | 4,3 | 0 | 49,2 |
| **Beispiel B2** | 5 | 4,8 | 82,2 | 497,9 | 5,5 | 0,44 | 83,6 |
| | 10 | 9,4 | 184,2 | 497,3 | 4,9 | 0,12 | 89,4 |
| | 20 | 21,0 | 363,8 | 498,6 | 4,4 | 0,04 | 58,1 |
| | 30 | 30,5 | 448,5 | 500,9 | 4,3 | 0,02 | 49,4 |
| **Beispiel C2** | 5 | 5,1 | 73,0 | 497,5 | 5,4 | 0,59 | 92,6 |
| | 10 | 10,3 | 172,5 | 501,5 | 4,9 | 0,13 | 94,6 |
| | 20 | 19,6 | 311,5 | 503,7 | 4,5 | 0,05 | 66,1 |
| | 30 | 31,2 | 401,2 | 504,8 | 4,4 | 0,03 | 52,0 |
| **Vergleich D2** | 5 | 5,3 | 35,7 | 498,1 | 5,6 | 2,51 | 87,3 |
| | 10 | 9,8 | 98,2 | 502,2 | 4,9 | 0,25 | 95,7 |
| | 20 | 20,8 | 181,8 | 504,5 | 4,5 | 0,07 | 66,9 |
| | 30 | 31,5 | 218,8 | 504,5 | 4,4 | 0,02 | 57,8 |
| **Vergleich E2** | 5 | 5,5 | 66,7 | 498,6 | 5,4 | 0,45 | 91,6 |
| | 10 | 10,1 | 139,1 | 501,2 | 4,9 | 0,13 | 94,1 |
| | 20 | 20,8 | 264,3 | 503,8 | 4,5 | 0,06 | 66,6 |
| | 30 | 28,8 | 304,7 | 502,5 | 4,4 | 0,02 | 60,0 |
| **Vergleich F2** | 5 | 4,9 | 26,1 | 493,6 | 5,6 | 7,70 | 74,5 |
| | 10 | 9,8 | 70,8 | 499,7 | 5,0 | 0,61 | 86,4 |
| | 20 | 20,7 | 149,1 | 501,5 | 4,5 | 0,16 | 65,5 |
| | 30 | 29,8 | 176,1 | 502,5 | 4,5 | 0,12 | 59,5 |
| **Vergleich G2** | 5 | 5,4 | 18,9 | 495,4 | 5,7 | 100,0 | 83,0 |
| | 10 | 9,8 | 45,4 | 502,2 | 5,1 | 1,57 | 90,8 |
| | 20 | 19,2 | 104,2 | 504,1 | 4,6 | 0,22 | 69,1 |
| | 30 | 29,8 | 126,5 | 506,1 | 4,5 | 0,14 | 59,0 |
| **Parteck® M200** | | 5,2 | 84,1 | 497,8 | 5,1 | 0,21 | 155,8 |
| | 10 | 10,7 | 196,5 | 500,6 | 4,6 | 0,17 | 306,0 |
| | | 20,3 | 340,0 | 499,4 | 4,2 | 0,15 | 513,6 |
| | | 30,0 | 396,7 | 498,3 | 4,0 | 0,16 | 647,6 |

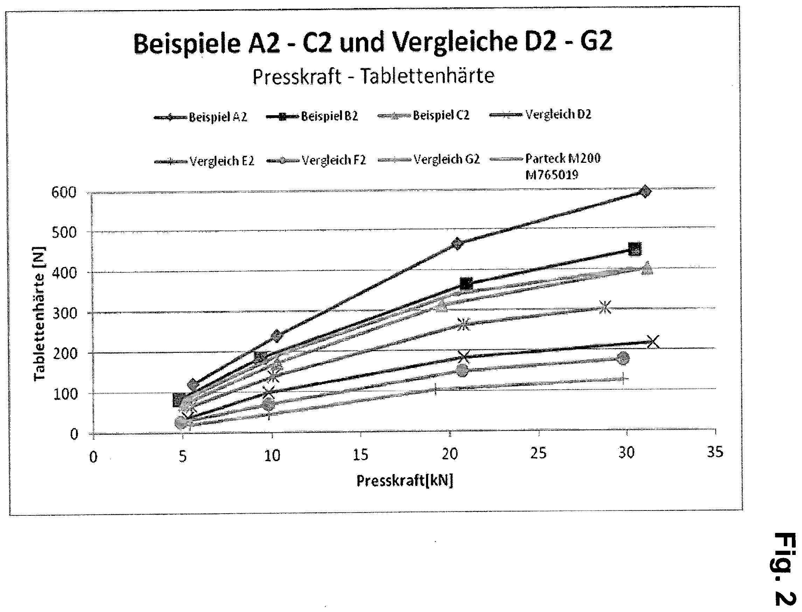

In der Figur 2 sind die stark unterschiedlichen Preßkraft-Tablettenhärte-Profile zur besseren Veranschaulichung grafisch dargestellt.

**3a. Herstellung der Abmischungen der direkt verpressbaren Trägerstoffe mit der gemahlenen PVA-Type 26-88**

Allgemeine Beschreibung: gemahlenes PVA 26-88 wird zur Entfernung von eventuellen Grobanteilen und Agglomeraten durch ein 800 μm Handsieb abgelegt. 300 g dieser gesiebten Ware werden in ein 2l Turbulamischgefäß eingewogen, 300 g der entsprechenden mikrokristallinen Cellulose aus Tabelle 3a hinzugegeben und 5 min. in einem Turbulamischer T2A gemischt.

**Tabelle 3a: Zusammensetzung der Co-Mischungen gemahlenes PVA 26-88 mit mikrokristallinen Cellulosen**

| Zusammensetzung | 50 Gew.-% PVA | 50 Gew.-% MCC |
|---|---|---|
| Beispiel A3 | PVA 26-88* | Avicel® PH 105 |
| Beispiel B3 | PVA 26-88* | Vivapur® 101 |
| Beispiel C3 | PVA 26-88* | Avicel® PH 101 |
| Vergleich D3 | PVA 26-88* | Avicel® PH 102 |
| Vergleich E3 | PVA 26-88* | Avicel® PH 102 SCG |
| Vergleich F3 | PVA 26-88* | Vivapur® 12 |
| Vergleich G3 | PVA 26-88* | Vivapur® 102 Premium |
| Vergleich H3 | PVA 26-88* | Vivapur® 200 |
| Vergleich I3 | PVA 26-88* | Emcocel® 90M |
| Vergleich J3 | PVA 26-88* | Emcocel® LP200 |
| Vergleich K3 | PVA 26-88* | Comprecel® M302 |
| *: vermahlenes PVA | | |

**3b. Verpressung dieser Abmischungen und Tablettencharakterisierung**

Allg. Beschreibung: je 498,75 g der oben hergestellten Co-Mischungen aus den Beispielen A3-C3 bzw. den Vergleichen D3-K3 werden in einem Turbulamischgefäß mit 1,25 g Magnesiumstearat versetzt, nochmals 5 min. in einem Turbulamischer T2A gemischt und an einer Exzenterpresse Korsch EK 0-DMS tablettiert.

Als Vergleich dient Parteck® M200 abgemischt mit 1% Parteck® LUB MST. Anmerkung: eine Verpressung des Parteck® M200 mit weniger Magnesiumstearat ist wegen der ansonsten resultierenden sehr hohen Ausstoßkräfte nicht möglich.

**Tabelle 3b: Tablettierdaten der Co-Mischungen gemahlenes PVA 26-88 mit mikrokristallinen Cellulosen**

Legende:

A: Presskraft [kN]          B: Tablettenhärte nach 1 Tag [N]
C: Tablettenmasse [mg]      D: Tablettenhöhe [mm]
E: Abrieb [%]              F: Ausstoßkraft(N)

| | A | | B | C | D | E | F |
|---|---|---|---|---|---|---|---|
| | Soll | Ist | | | | | |
| **Beispiel A3** | 5 | 5,1 | 104,9 | 487,9 | 5,2 | 0,08 | 97,2 |
| | 10 | 9,0 | 190,6 | 481,6 | 4,8 | 0 | 102,7 |
| | 20 | 17,3 | 350,8 | 476,0 | 4,3 | 0 | 69,7 |
| | 30 | 27,2 | 469,7 | 473,4 | 4,1 | 0 | 43,2 |
| **Beispiel B3** | 5 | 4,9 | 93,5 | 497,9 | 5,5 | 0,33 | 98,1 |
| | 10 | 10,6 | 221,4 | 500,0 | 4,8 | 0,09 | 99,3 |
| | 20 | 20,5 | 408,6 | 503,0 | 4,4 | 0,02 | 62,8 |
| | 30 | 30,6 | 492,3 | 503,4 | 4,3 | 0,03 | 51,7 |
| **Beispiel C3** | 5 | 4,7 | 79,9 | 496,6 | 5,5 | 0,37 | 93,6 |
| | 10 | 10,5 | 201,5 | 500,1 | 4,8 | 0,05 | 93,4 |
| | 20 | 19,6 | 348,8 | 503,2 | 4,5 | 0 | 58,5 |
| | 30 | 31,3 | 424,1 | 502,9 | 4,4 | 0 | 44,8 |
| **Vergleich D3** | 5 | 4,9 | 70,2 | 501,8 | 5,4 | 0,49 | 85,9 |
| | 10 | 9,6 | 153,1 | 506,1 | 4,9 | 0,16 | 87,3 |

(fortgesetzt)

| Legende: | | | | | | | |
|---|---|---|---|---|---|---|---|
| A: Presskraft [kN] | | | B: Tablettenhärte nach 1 Tag [N] | | | | |
| C: Tablettenmasse [mg] | | | D: Tablettenhöhe [mm] | | | | |
| E: Abrieb [%] | | | F: Ausstoßkraft(N) | | | | |

| | A | | B | C | D | E | F |
|---|---|---|---|---|---|---|---|
| | Soll | Ist | | | | | |
| | 20 | 18,4 | 267,3 | 506,6 | 4,5 | 0,07 | 61,1 |
| | 30 | 28,6 | 325,1 | 506,8 | 4,4 | 0,04 | 52,1 |
| **Vergleich E3** | 5 | 5,1 | 50,4 | 495,5 | 5,4 | 1,18 | 80,1 |
| | 10 | 9,7 | 106,3 | 499,2 | 4,8 | 0,38 | 80,9 |
| | 20 | 18,8 | 180,1 | 499,6 | 4,5 | 0,21 | 60,3 |
| | 30 | 30,2 | 209,6 | 499,7 | 4,4 | 0,16 | 55,4 |
| **Vergleich F3** | 5 | 4,8 | 47,6 | 496,3 | 5,6 | 1,52 | 95,3 |
| | 10 | 10,2 | 134,0 | 501,1 | 4,9 | 0,16 | 105,2 |
| | 20 | 20,7 | 251,4 | 502,9 | 4,5 | 0,06 | 75,5 |
| | 30 | 31,6 | 299,4 | 503,7 | 4,4 | 0,03 | 66,2 |
| **Vergleich G** | 5 | 5,2 | 70,2 | 497,8 | 5,5 | 0,39 | 87,9 |
| | 10 | 9,8 | 146,5 | 498,1 | 4,9 | 0,08 | 92,4 |
| | 20 | 19,8 | 273,1 | 499,8 | 4,5 | 0,01 | 66,2 |
| | 30 | 30,8 | 331,8 | 499,9 | 4,4 | 0 | 56,8 |
| **Vergleich G3** | 5 | 5,1 | 76,8 | 498,4 | 5,4 | 0,26 | 91,3 |
| | 10 | 10,2 | 171,4 | 502,1 | 4,8 | 0,05 | 91,8 |
| | 20 | 19,5 | 295,7 | 503,4 | 4,5 | 0 | 66,7 |
| | 30 | 30,0 | 354,5 | 502,5 | 4,4 | 0 | 58,6 |
| **Vergleich H3** | 5 | 4,8 | 41,8 | 498,4 | 5,5 | 1,89 | 88,5 |
| | 10 | 9,8 | 113,0 | 502,7 | 4,9 | 0,29 | 96,4 |
| | 20 | 20,5 | 213,8 | 502,1 | 4,4 | 0,09 | 70,0 |
| | 30 | 30,4 | 244,2 | 502,6 | 4,4 | 0,07 | 64,2 |
| **Vergleich I3** | 5 | 4,9 | 71,0 | 494,2 | 5,5 | 0,39 | 90,9 |
| | 10 | 10,2 | 159,6 | 497,0 | 4,9 | 0,06 | 92,3 |
| | 20 | 20,0 | 273,6 | 496,8 | 4,5 | 0 | 64,8 |
| | 30 | 30,4 | 318,0 | 498,2 | 4,4 | 0 | 57,3 |
| **Vergleich J3** | 5 | 5,1 | 28,6 | 494,9 | 5,5 | 5,64 | 93,4 |
| | 10 | 10,0 | 78,7 | 499,2 | 4,9 | 0,46 | 97,3 |
| | 20 | 20,3 | 144,7 | 501,0 | 4,5 | 0,15 | 70,7 |
| | 30 | 29,6 | 161,2 | 501,9 | 4,4 | 0,12 | 63,9 |
| **Vergleich K3** | 5 | 5,1 | 39,8 | 497,6 | 5,5 | 1,50 | 90,4 |
| | 10 | 10,2 | 100,6 | 499,1 | 4,9 | 0,16 | 93,6 |
| | 20 | 19,0 | 184,2 | 500,1 | 4,5 | 0,03 | 71,6 |
| | 30 | 30,7 | 224,2 | 500,6 | 4,4 | 0,02 | 62,3 |
| **Parteck® M200** | 5 | 5,2 | 84,1 | 497,8 | 5,1 | 0,21 | 155,8 |
| | 10 | 10,7 | 196,5 | 500,6 | 4,6 | 0,17 | 306,0 |
| | 20 | 20,3 | 340,0 | 499,4 | 4,2 | 0,15 | 513,6 |
| | 30 | | 396,7 | 498,3 | 4,0 | 0,16 | 647,6 |

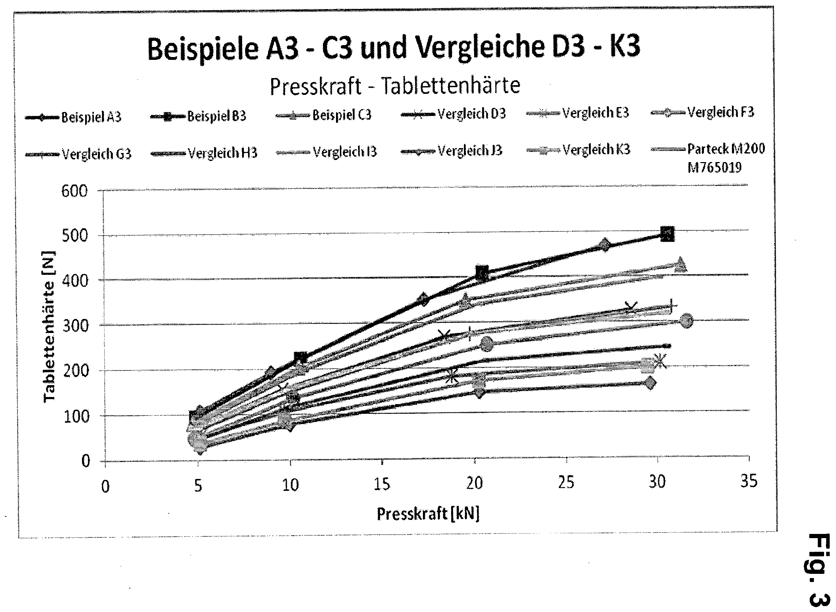

In der Figur 3 sind die stark unterschiedlichen Preßkraft-Tablettenhärte-Profile zur besseren Veranschaulichung grafisch dargestellt.

**4a. Herstellung der Abmischungen der direkt verpressbaren Trägerstoffe mit der gemahlenen PVA-Type 40-88**

Allgemeine Beschreibung: gemahlenes PVA 40-88 wird zur Entfernung von eventuellen Grobanteilen und Agglomeraten durch ein 800 μm Handsieb abgelegt. 300 g dieser gesiebten Ware werden in ein 2l Turbulamischgefäß eingewogen, 300 g der entsprechenden mikrokristallinen Cellulose aus Tabelle 4a hinzugegeben und 5 min. in einem Turbulamischer T2A gemischt.

**Tabelle 4a: Zusammensetzung der Co-Mischungen gemahlenes PVA 40-88 mit mikrokristallinen Cellulosen**

| Zusammensetzung | 50 Gew.-% PVA | 50 Gew.-% MCC |
|---|---|---|
| Beispiel A4 | PVA 40-88* | Avicel® PH 105 |
| Beispiel B4 | PVA 40-88* | Vivapur® 101 |
| Beispiel C4 | PVA 40-88* | Avicel® PH 101 |
| Vergleich D4 | PVA 40-88* | Vivapur® 12 |
| Vergleich E4 | PVA 40-88* | Vivapur® 102 Premium |
| Vergleich F4 | PVA 40-88* | Vivapur® 200 |
| Vergleich G4 | PVA 40-88* | Emcocel® LP200 |
| *: vermahlenes PVA | | |

**4b. Verpressung dieser Abmischungen und Tablettencharakterisierung**

Allg. Beschreibung: je 498,75 g der oben hergestellten Co-Mischungen aus den Beispielen A4-C4 bzw. den Vergleichen D4-G4 werden in einem Turbulamischgefäß mit 1,25 g Magnesiumstearat versetzt, nochmals 5 min. in einem Turbulamischer T2A gemischt und an einer Exzenterpresse Korsch EK 0-DMS tablettiert.

Als Vergleich dient Parteck® M200 abgemischt mit 1% Parteck® LUB MST. Anmerkung: eine Verpressung des Parteck® M200 mit weniger Magnesiumstearat ist wegen der sonst erforderlichen sehr hohen Ausstoßkräfte nicht möglich.

**Tabelle 4b: Tablettierdaten der Co-Mischungen gemahlenes PVA 40-88 mit mikrokristallinen Cellulosen**

Legende:

A: Presskraft [kN]  B: Tablettenhärte nach 1 Tag [N]
C: Tablettenmasse [mg]  D: Tablettenhöhe [mm]
E: Abrieb [%]  F: Ausstoßkraft(N)

| | A | | B | C | D | E | F |
| | Soll | Ist | | | | | |
|---|---|---|---|---|---|---|---|
| **Beispiel A4** | 5 | 5,4 | 110,8 | 488,7 | 5,3 | 0,11 | 100,3 |
| | 10 | 10,4 | 235,6 | 488,4 | 4,7 | 0 | 97,4 |
| | 20 | 23,1 | 462,7 | 481,9 | 4,3 | 0 | 53,0 |
| | 30 | 29,5 | 546,4 | 485,6 | 4,2 | 0 | 44,2 |
| **Beispiel B4** | 5 | 5,1 | 88,3 | 495,9 | 5,3 | 0,41 | 82,9 |
| | 10 | 10,6 | 203,5 | 496,2 | 4,7 | 0,13 | 81,8 |
| | 20 | 19,7 | 352,1 | 501,1 | 4,4 | 0,06 | 56,7 |
| | 30 | 28,8 | 414,9 | 504,0 | 4,4 | 0,07 | 47,5 |
| **Beispiel C4** | 5 | 5,1 | 74,2 | 499,8 | 5,5 | 0,57 | 85,9 |
| | 10 | 9,6 | 152,8 | 501,6 | 5,0 | 0,19 | 87,9 |
| | 20 | 19,4 | 289,0 | 503,2 | 4,5 | 0,06 | 58,1 |
| | 30 | 29,7 | 358,2 | 503,8 | 4,4 | 0,07 | 47,5 |

(fortgesetzt)

| | A | | B | C | D | E | F |
|---|---|---|---|---|---|---|---|
| | Soll | Ist | | | | | |
| **Vergleich D4** | 5 | 5,0 | 35,7 | 497,3 | 5,5 | 2,89 | 81,7 |
| | 10 | 10,0 | 87,3 | 502,0 | 4,9 | 0,32 | 91,4 |
| | 20 | 20,7 | 172,5 | 502,4 | 4,5 | 0,11 | 67,6 |
| | 30 | 30,3 | 205,5 | 504,9 | 4,4 | 0,05 | 59,4 |
| **Vergleich E4** | 5 | 5,0 | 64,2 | 500,4 | 5,4 | 0,49 | 86,8 |
| | 10 | 10,3 | 146,9 | 505,7 | 4,9 | 0,15 | 87,3 |
| | 20 | 20,1 | 247,4 | 506,0 | 4,5 | 0,08 | 62,5 |
| | 30 | 32,0 | 296,6 | 506,0 | 4,5 | 0,07 | 55,9 |
| **Vergleich F4** | 5 | 5,2 | 32,9 | 497,1 | 5,5 | 3,16 | 72,9 |
| | 10 | 10,4 | 82,3 | 500,8 | 4,8 | 0,43 | 79,2 |
| | 20 | 19,6 | 149,2 | 501,2 | 4,4 | 0,18 | 60,9 |
| | 30 | 30,9 | 180,2 | 502,7 | 4,4 | 0,12 | 54,8 |
| **Vergleich G4** | 5 | 5,2 | 19,4 | 491,0 | 5,5 | 100,0 | 75,3 |
| | 10 | 10,0 | 45,7 | 498,5 | 5,0 | 1,26 | 80,0 |
| | 20 | 20,2 | 92,7 | 500,4 | 4,6 | 0,33 | 59,3 |
| | 30 | 31,0 | 105,9 | 501,9 | 4,5 | 0,26 | 52,6 |
| **Parteck® M200** | 5 | 5,2 | 84,1 | 497,8 | 5,1 | 0,21 | 155,8 |
| | 10 | 10,7 | 196,5 | 500,6 | 4,6 | 0,17 | 306,0 |
| | 20 | 20,3 | 340,0 | 499,4 | 4,2 | 0,15 | 513,6 |
| | 30 | 30,0 | 396,7 | 498,3 | 4,0 | 0,16 | 647,6 |

Legende:

A: Presskraft [kN]    B: Tablettenhärte nach 1 Tag [N]
C: Tablettenmasse [mg]    D: Tablettenhöhe [mm]
E: Abrieb [%]    F: Ausstoßkraft(N)

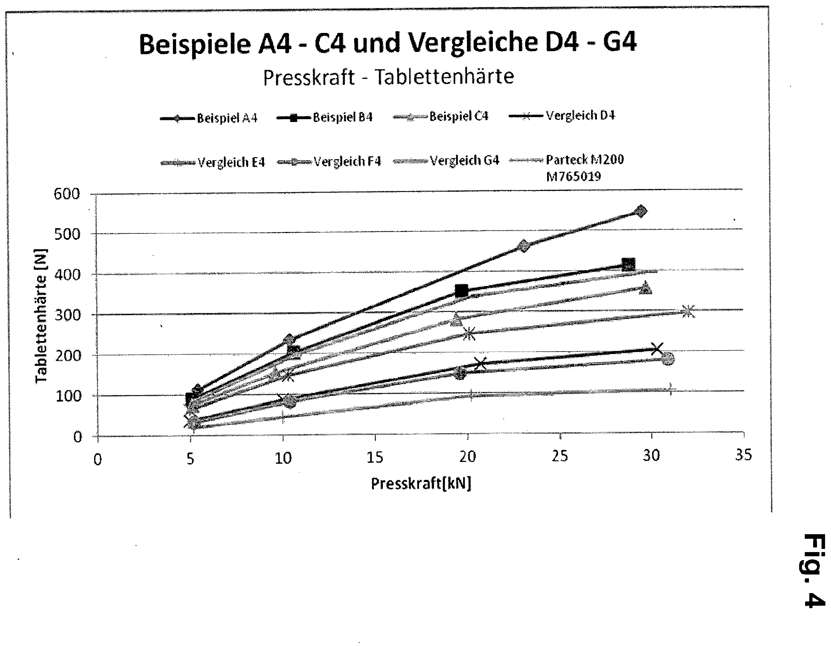

In der Figur 4 sind die stark unterschiedlichen Preßkraft-Tablettenhärte-Profile zur besseren Veranschaulichung graphisch dargestellt.

**Patentansprüche**

1.    Direkt verpressbare Co-Mischungen, enthaltend feinkörnige Polyvinylalkohole (PVAs) mit einer mittleren Partikelgröße $D_{v50}$ im Bereich von 50 bis 260 $\mu$m und feinkörnige mikrokristalline Cellulosen (MCCs) mit mittleren Korngrößen von $D_{v50} < 70$ $\mu$m, besonders bevorzugt mit mittleren Korngrößen im Bereich von $D_{v50}$ 17 bis 67 $\mu$m, insbesondere im Bereich von $D_{v50}$ 17 $\mu$m - 20 $\mu$m.

2.    Direkt verpressbare Co-Mischungen gemäß Anspruch 1, enthaltend feinkörnige Polyvinylalkohole zu feinkörnigen mikrokristallinen Cellulosen im Verhältnis von 5 : 1 bis 1 : 5, bevorzugt 2 : 1 bis 1 :2, insbesondere bevorzugt im Verhältnis von 1 : 1 bezogen auf das Gewicht.

3.    Direkt verpressbare Co-Mischungen gemäß Anspruch 1 oder 2, enthaltend feinkörnige Polyvinylalkohole, welche die Anforderungen der Pharmakopöen (Ph. Eur., USP und JPE) erfüllen und die zur Wirkstoffretardierung geeignet sind.

4.    Direkt verpressbare Co-Mischungen gemäß einem oder mehreren der Ansprüche 1, 2 oder 3, enthaltend feinkörnige Polyvinylalkohole der Typen 4-88, 18-88, 26-88 und 40-88, die den Anforderungen der Pharmakopöen Ph. Eur., JPE und der USP, sowie Type 28-99, die den Pharmakopöen JPE und Ph. Eur. entsprechen.

5.  Direkt verpressbare Co-Mischungen gemäß einem oder mehreren der Ansprüche 1 bis 4, enthaltend feinkörnige Polyvinylalkohole (PVAs), welche der Ph. Eur. entsprechen und welche durch Polymerisation von Vinylacetat und durch anschließende teilweise oder nahezu vollständige Hydrolyse des Polyvinylacetats erhalten worden sind und eine durchschnittliche relative Molekülmasse im Bereich zwischen 20 000 und 150 000 g/mol haben, und die nach Ph. Eur. eine Viskosität im Bereich von 3 - 70 mPa.s, (gemessen in einer 4%igen Lösung bei 20 °C) besitzen und eine Esterzahl von nicht größer als 280 mg KOH/g (Hydrolysegrad >72.2 mol%) aufweisen.

6.  Direkt verpressbare Co-Mischungen gemäß einem oder mehreren der Ansprüche 1 bis 5, enthaltend feinkörnige Polyvinylalkohole (PVAs), welche der USP entsprechen und als in Wasser lösliche, synthetische Harze vorliegen, die durch die Formel

$$(C_2H_4O)_n$$

charakterisiert sind, worin

n eine ganze Zahl im Bereich von 500 und bis 5 000 bedeutet,
und welche durch 85 - 89 %ige Hydrolyse des Polyvinylacetats erhalten worden sind.

7.  Wirkstoffhaltige Tabletten mit verlängerter Wirkstofffreisetzung, enthaltend feinkörnige Polyvinylalkohole (PVAs) mit einer mittleren Partikelgröße $D_{v50}$ im Bereich von 50 bis 260 $\mu$m und feinkörnige mikrokristalline Cellulosen (MCCs) mit mittleren Korngrößen von $D_{v50}$<70 $\mu$m, besonders bevorzugt mit mittleren Korngrößen im Bereich von $D_{v50}$ 17 bis 67 $\mu$m, insbesondere im Bereich von $D_{v50}$ 17 $\mu$m - 20 $\mu$m.

8.  Wirkstoffhaltige Tabletten mit verlängerter Wirkstofffreisetzung von mehreren Stunden, enthaltend eine Co-Mischung aus feinkörnigen Polyvinylalkoholen (PVAs)und feinkörnigen mikrokristallinen Cellulosen (MCCs) gemäß einem oder mehreren der Ansprüche 1 bis 6.

9.  Wirkstoffhaltige Tabletten gemäß Anspruch 7 oder 8, enthaltend eine direkt verpressbare Co-Mischung gemäß einem oder mehreren der Ansprüche 1 bis 6 in einer Menge im Bereich von 1 - 99 Gew.-%, bevorzugt in einer Menge von 5 - 95 Gew.-%, ganz besonders bevorzugt in einer Menge von 10 - 90 Gew.-% bezogen auf das Gesamtgewicht der Tablette.

10.  Wirkstoffhaltige Tabletten gemäß einem oder mehreren der Ansprüche 7 bis 9, welche bei Herstellung unter Einsatz von niedrigen Presskräften Tabletten mit besonders hohen Tablettenhärten und geringen Friabilitäten von ≤0.2 Gew.-% liefern, wobei aber nur geringe Ausstoßkräfte aufzuwenden sind

11.  Wirkstoffhaltige Tabletten gemäß einem oder mehreren der Ansprüche 7 bis 10 mit verzögerter Wirkstofffreisetzung, enthaltend Wirkstoffe der BCS Klasse I entweder allein oder in Kombination mit anderen Wirkstoffen.

12.  Verwendung von direkt verpressbaren Co-Mischungen gemäß einem oder mehreren der Ansprüche 1 - 6 zur Herstellung von Tabletten, wobei durch Kompression mit einer Presskraft von 10 kN Tabletten mit Härten von ≥153 N erhalten werden mit einer Friabilität von ≤0.2 Gew.-%.

13.  Verwendung von direkt verpressbaren Co-Mischungen gemäß einem oder mehreren der Ansprüche 1 - 6 zur Herstellung von Tabletten, wobei durch Kompression mit einer Presskraft von 20 kN Tabletten mit Härten von ≥289 N erhalten werden mit einer Friabilität von ≤0.1 Gew.-%.

**Claims**

1.  Directly compressible co-mixtures comprising fine-grained polyvinyl alcohols (PVAs) having an average particle size $D_{v50}$ in the range from 50 to 260 $\mu$m and fine-grained microcrystalline celluloses (MCCs) having average particle sizes of $D_{v50}$<70 $\mu$m, particularly preferably having average particle sizes in the range from $D_{v50}$ 17 to 67 $\mu$m, in particular in the range from $D_{v50}$ 17 $\mu$m - 20 $\mu$m.

2.  Directly compressible co-mixtures according to Claim 1, comprising fine-grained polyvinyl alcohols to fine-grained microcrystalline celluloses in the ratio from 5 : 1 to 1 : 5, preferably 2 : 1 to 1 : 2, particularly preferably in the ratio of 1 : 1, based on the weight.

3. Directly compressible co-mixtures according to Claim 1 or 2, comprising fine-grained polyvinyl alcohols which meet the requirements of the pharmacopoeias (Ph. Eur., USP and JPE) and which are suitable for retardation of active compound.

4. Directly compressible co-mixtures according to one or more of Claims 1, 2 and 3, comprising fine-grained polyvinyl alcohols of grades 4-88, 18-88, 26-88 and 40-88 which meet the requirements of the pharmacopoeias Ph. Eur., JPE and USP, and grade 28-99, which conforms to the pharmacopoeias JPE and Ph. Eur.

5. Directly compressible co-mixtures according to one or more of Claims 1 to 4, comprising fine-grained polyvinyl alcohols (PVAs) which conform to Ph. Eur. and which have been obtained by polymerisation of vinyl acetate and by subsequent partial or virtually complete hydrolysis of the polyvinyl acetate and have an average relative molecular weight in the range between 20,000 and 150,000 g/mol, and which have a viscosity in accordance with Ph. Eur. in the range 3-70 mPa.s (measured in a 4% solution at 20°C) and have an ester value of not greater than 280 mg of KOH/g (degree of hydrolysis >72.2 mol%)..

6. Directly compressible co-mixtures according to one or more of Claims 1 to 5, comprising fine-grained polyvinyl alcohols (PVAs) which conform to the USP and are in the form of water-soluble, synthetic resins which are **characterised by** the formula

$$(C_2H_4O)_n$$

in which

n denotes an integer in the range from 500 to 5000,
and which have been obtained by 85 - 89% hydrolysis of the polyvinyl acetate.

7. Active-compound-containing tablets having extended release of active compound, comprising fine-grained polyvinyl alcohols (PVAs) having an average particle size $D_{v50}$ in the range from 50 to 260 $\mu$m and fine-grained microcrystalline celluloses (MCCs) having average particle sizes of $D_{v50}$<70 $\mu$m, particularly preferably having average particle sizes in the range from $D_{v50}$ 17 to 67 $\mu$m, in particular in the range from $D_{v50}$ 17 $\mu$m - 20 $\mu$m.

8. Active-compound-containing tablets having extended release of active compound of several hours, comprising a co-mixture of fine-grained polyvinyl alcohols (PVAs) and fine-grained microcrystalline celluloses (MCCs) according to one or more of Claims 1 to 6.

9. Active-compound-containing tablets according to Claim 7 or 8, comprising a directly compressible co-mixture according to one or more of Claims 1 to 6 in an amount in the range 1 - 99% by weight, preferably in an amount of 5 - 95% by weight, very particularly preferably in an amount of 10 - 90% by weight, based on the total weight of the tablet.

10. Active-compound-containing tablets according to one or more of Claims 7 to 9, which, in the case of production using low pressing forces, give tablets having particularly high tablet hardnesses and low friabilities of ≤0.2 % by weight, but where only low ejection forces have to be used.

11. Active-compound-containing tablets according to one or more of Claims 7 to 10 having delayed release of active compound, comprising active compounds from BCS class I, either alone or in combination with other active compounds.

12. Use of directly compressible co-mixtures according to one or more of Claims 1 - 6 for the production of tablets, where tablets having hardnesses of ≥153 N with a friability of ≤0.2% by weight are obtained by compression with a pressing force of 10 kN.

13. Use of directly compressible co-mixtures according to one or more of Claims 1 - 6 for the production of tablets, where tablets having hardnesses of ≥289 N with a friability of ≤0.1% by weight are obtained by compression with a pressing force of 20 kN.

**Revendications**

1. Co-mélanges pouvant être directement comprimés, comprenant des alcools polyvinyliques (PVA) à grains fins qui présentent une taille de particule moyenne $D_{v50}$ dans la plage qui va de 50 $\mu$m à 260 $\mu$m et des celluloses micro-cristallines (MCC) à grains fins qui présentent une taille de particule moyenne $D_{v50}$ < 70 $\mu$m, de façon particulièrement préférable, qui présentent des tailles de particule moyennes $D_{v50}$ dans la plage qui va de 17 $\mu$m à 67 $\mu$m, en particulier, dans la plage de $D_{v50}$ qui va de 17 $\mu$m à 20 $\mu$m.

2. Co-mélanges pouvant être directement comprimés selon la revendication 1, présentant un rapport de la quantité des alcools polyvinyliques à grains fins sur la quantité des celluloses microcristallines à grains fins qui est égal au rapport qui va de 5 : 1 à 1 : 5, de préférence, qui va de 2 : 1 à 1 : 2, de façon particulièrement préférable, qui est égal au rapport de 1 : 1, sur la base du poids.

3. Co-mélanges pouvant être directement comprimés selon la revendication 1 ou 2, comprenant des alcools polyvinyliques à grains fins qui satisfont les exigences des pharmacopées (Ph. Eur., USP et JPE) et qui conviennent pour le retardement du composé actif.

4. Co-mélanges pouvant être directement comprimés selon l'une des revendications 1, 2 et 3, comprenant des alcools polyvinyliques à grains fins des classes 4-88, 18-88, 26-88 et 40-88 qui satisfont les exigences des pharmacopées Ph. Eur., JPE et USP, et de la classe 28-99, laquelle est conforme aux pharmacopées JPE et Ph. Eur.

5. Co-mélanges pouvant être directement comprimés selon une ou plusieurs des revendications 1 à 4, comprenant des alcools polyvinyliques (PVA) à grains fins qui sont conformes à la pharmacopée Ph. Eur. et qui ont été obtenus au moyen de la polymérisation d'acétate de vinyle et au moyen de l'hydrolyse partielle ou virtuellement complète subséquente de l'acétate de polyvinyle et qui présentent un poids moléculaire relatif moyen dans la plage entre 20 000 g/mol et 150 000 g/mol, et qui présentent une viscosité conformément à la pharmacopée Ph. Eur. dans la plage qui va de 3 mPa.s à 70 mPa.s (telle que mesurée dans une solution à 4% à 20°C) et qui présentent un indice d'ester non supérieur à 280 mg de KOH/g (degré d'hydrolyse > 72,2 % en pourcentage molaire).

6. Co-mélanges pouvant être directement comprimés selon une ou plusieurs des revendications 1 à 5, comprenant des alcools polyvinyliques (PVA) à grains fins qui sont conformes à la pharmacopée USP et qui se présentent sous la forme de résines synthétiques solubles à l'eau qui sont **caractérisées par** la formule :

$$(C_2H_4O)_n$$

dans laquelle :

n représente un entier dans la plage qui va de 500 à 5000,
et qui ont été obtenus au moyen d'une hydrolyse à 85 % - 89% de l'acétate de polyvinyle.

7. Comprimés contenant un composé actif qui présentent une libération prolongée du composé actif, comprenant des alcools polyvinyliques (PVA) à grains fins qui présentent une taille de particule moyenne $D_{v50}$ dans la plage qui va de 50 $\mu$m à 260 $\mu$m et des celluloses microcristallines (MCC) à grains fins qui présentent des tailles de particule moyennes $D_{v50}$ < 70 $\mu$m, de façon particulièrement préférable, qui présentent des tailles de particule moyennes dans la plage de $D_{v50}$ qui va de 17 $\mu$m à 67 $\mu$m, en particulier dans la plage de $D_{v50}$ qui va de 17 $\mu$m à 20 $\mu$m.

8. Comprimés contenant un composé actif qui présentent une libération prolongée du composé actif de plusieurs heures, comprenant un co-mélange d'alcools polyvinyliques (PVA) à grains fins et de celluloses microcristallines (MCC) à grains fins selon une ou plusieurs des revendications 1 à 6.

9. Comprimés contenant un composé actif selon la revendication 7 ou 8, comprenant un co-mélange pouvant être directement comprimé selon une ou plusieurs des revendications 1 à 6 selon une quantité dans la plage qui va de 1 % à 99 % en poids, de préférence, selon une quantité qui va de 5 % à 95 % en poids, de façon très particulièrement préférable, selon une quantité qui va de 10 % à 90 % en poids, sur la base du poids total du comprimé.

10. Comprimés contenant un composé actif selon une ou plusieurs des revendications 7 à 9, lesquels, dans le cas d'une fabrication en utilisant des forces de pression faibles, permettent d'obtenir des comprimés qui présentent des duretés de comprimé particulièrement élevées et des friabilités particulièrement faibles ≤ 0,2 % en poids, mais pour lesquels seulement des forces d'éjection faibles doivent être utilisées.

11. Comprimés contenant un composé actif selon une ou plusieurs des revendications 7 à 10 qui présentent une libération retardée du composé actif, comprenant des composés actifs pris parmi la classe BCS I, soit seuls, soit en combinaison avec d'autres composés actifs.

12. Utilisation de co-mélanges pouvant être directement comprimés selon une ou plusieurs des revendications 1 à 6 pour la fabrication de comprimés, dans laquelle des comprimés présentant des duretés $\geq$ 153 N avec une friabilité $\leq$ 0,2% en poids sont obtenus par compression selon une force de pression de 10 kN.

13. Utilisation de co-mélanges pouvant être directement comprimés selon une ou plusieurs des revendications 1 à 6 pour la fabrication de comprimés, dans laquelle des comprimés présentant des duretés $\geq$ 289 N avec une friabilité $\leq$ 0,1% en poids sont obtenus par compression selon une force de pression de 20 kN.

EP 3 174 531 B1

**Beispiele A1 - C1 und Vergleiche D1 - G1**

Presskraft - Tablettenhärte

**Fig. 1**

Fig. 2

**Beispiele A2 - C2 und Vergleiche D2 - G2**
Presskraft - Tablettenhärte

## Beispiele A3 - C3 und Vergleiche D3 - K3

### Presskraft - Tablettenhärte

─◇─Beispiel A3　　─■─Beispiel B3　　─▲─Beispiel C3　　─✕─Vergleich D3　　─✳─Vergleich E3　　─○─Vergleich F3

─┼─Vergleich G3　　──Vergleich H3　　──Vergleich I3　　─▽─Vergleich J3　　──Vergleich K3　　──Parteck M200 M765019

EP 3 174 531 B1

**Fig. 3**

**Beispiele A4 - C4 und Vergleiche D4 - G4**

Presskraft - Tablettenhärte

EP 3 174 531 B1

**Fig. 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2008152595 A1

**In der Beschreibung aufgeführte Nicht-Patentliteratur**

- **AMIDON GL ; LENNERNAS H ; SHAH VP ; CRISON JR.** A theoretical basis for a biopharmaceutic drug classification: the correlation of in vitro product dissolution and in vivo bioavailability. *Pharm Res.,* 1995, vol. 12, 413

- **VON S.BRUNAUER et al.** BET Surface Area by Nitrogen Absorption. *Journal of American Chemical Society,* 1983, vol. 60, 9